# EUROPEAN PATENT APPLICATION

(11) **EP 4 722 344 A1**
(43) Date of publication of application: **08.04.2026**
(21) Application number: 25773050.7
(22) Date of filing: 18.03.2025
(51) Int. Cl.: C12N 5/071, C12N 5/074, G01N 33/50, C12R 1/91

(54) **IMMORTALIZED HUMANIZED SEBACEOUS GLAND PRECURSOR CELL, PREPARATION METHOD THEREFOR, AND METHOD FOR SCREENING AND EVALUATING GREASE REGULATION DRUG OR SKIN CARE PRODUCT BY USING SEBACEOUS GLAND PRECURSOR CELL**

(30) Priority: 22.03.2024 CN 202410332472
(71) Applicant: Institute of Basic Theory for Chinese Medicine, China Academy of Chinese Medical Sciences, Beijing 100000 (CN); Beijing Uproven Medical Technology Co.Ltd, Beijing 100000 (CN)
(72) Inventor: XU, Ruodan, Beijing 100000 (CN); LI, Ning, Beijing 100000 (CN); SONG, Ping, Beijing 100000 (CN); SUN, Yu, Beijing 100000 (CN); QIU, Wei, Beijing 100000 (CN); CHEN, Yong, Beijing 100000 (CN); LIU, Youting, Beijing 100000 (CN)
(74) Representative: Bayramoglu et al.
(86) International application number: PCT/CN2025/083066
(87) International publication number: WO 2025/195351

(57) **Abstract**

The present disclosure provides an immortalized human sebaceous gland progenitor cell deposited at the China Center for Type Culture Collection (CCTCC) under an accession number of CCTCC NO: C2023286. The present disclosure also provides a preparation method and use of the immortalized human sebaceous gland progenitor cell. The immortalized human sebaceous gland progenitor cell can be directly used for screening and evaluating oil-regulating drugs or skincare products. Alternatively, the immortalized human sebaceous gland progenitor cell can further differentiate into a mature sebaceous gland cell, and the mature sebaceous gland cell is then utilized for screening and evaluating oil-regulating drugs or skincare products. The present disclosure has the following advantage: The sebaceous gland progenitor cell and the mature sebaceous gland cell are stable and accurate, and the expression of genes related to liquid droplet formation, lipid storage, and androgen receptor (AR) in these two cells is significantly enhanced. As a result, these two cells are highly suitable for screening and evaluation of oil-regulating drugs or skincare products.

## Description

### TECHNICAL FIELD

The present disclosure belongs to the technical field of skin oil-regulating models, and specifically relates to an immortalized human sebaceous gland progenitor cell, a preparation method thereof, and a method for screening and evaluating oil-regulating drugs or skincare products using the same.

### BACKGROUND TECHNOLOGY

In recent years, the incidence of cosmetologically-problematic skin diseases such as acne vulgaris, xeroderma, and seborrheic dermatitis has significantly increased. The pathogenesis of these skin diseases is closely associated with the dysfunctions of sebaceous glands. Sebaceous glands, located in the dermis, are one of the essential accessory organs of the skin. There are sebaceous gland cells in sebaceous glands. Newly-generated sebaceous gland cells continuously migrate toward the center, and grow in volume. During this process, liquid droplets within the cytoplasm accumulate progressively, the nucleus undergoes pyknosis, organelles disappear, and the cytoplasm becomes filled with liquid droplets. Finally, the sebaceous gland cells disintegrate and are expelled along with the liquid droplets, resulting in sebum. The secreted sebum can moisturize the skin and hair. The secreted sebum, together with sweat, forms a lipid film to protect the skin, which plays a crucial role in maintaining skin homeostasis. Thus, the dysfunction of sebaceous glands can lead to various skin problems.

Acne is a chronic inflammatory skin condition involving hair follicles and sebaceous glands. Acne predominantly occurs on the face, chest, and back. Clinically, acne is primarily manifested as comedones, papules, pustules, cysts, or nodules, and is often accompanied by enlarged pores and seborrhea. The incidence of acne in adolescents is as high as 93%. Acne has become the eighth most common chronic disease globally. A cross-sectional study indicates an acne morbidity of 8.1% in the Chinese population. Studies have shown that more than 95% of people will experience acne to varying degrees. Without early intervention and treatment, acne can easily lead to scarring, which severely affects the life quality of an acne patient. Therefore, to elucidate pathophysiological mechanisms of acne and develop pharmaceuticals or cosmetics for inhibiting the abnormal sebum secretion, it is crucial to construct a cell model capable of replicating behaviors of sebaceous glands in acne patients.

Currently, a hamster sebaceous gland cell line is known as a sebaceous gland cell line that can be used in experiments. This hamster sebaceous gland cell line shares the ability of forming liquid droplets within cells with sebaceous gland cells in living organisms. However, the genome information of this hamster sebaceous gland cell line remains unclear, making this hamster sebaceous gland cell line unsuitable for research employing genetic approaches. Non-patent document 1 discloses a human sebaceous gland cell line SZ95 immortalized through the transfection of large T antigen DNA of simian virus 40 (SV40), which is also commonly used in sebaceous gland-related experimental studies. This human sebaceous gland cell line is derived from the human, and has complete and clear genome information and a high proliferative capacity. However, the human sebaceous gland cell line SZ95 lacks androgen receptors (ARs), and cannot respond to testosterone to promote lipogenesis. When cultured *in vitro,* this human sebaceous gland cell line loses the ability to form liquid droplets. As a result, this human sebaceous gland cell line cannot fully represent sebaceous gland cells at a natural growth state, let alone replicate the behaviors of sebaceous glands in acne patients. Moreover, due to the introduction of oncogenes from human papillomavirus (HPV) and Epstein-Barr virus (EBV), the target cells may have a potential risk of malignant transformation (Zouboulis CC, Seltmann H, Neitzel H, et al. Establishment and characterization of an immortalized human sebaceous gland cell line (sz95) [J]. J Invest Dermatol, 1999, 113 (6): 1011-1020). Non-patent document 2 discloses interventions with different vegetable oils to induce the lipidation in SZ95 cells. Although this method can stimulate the generation of sebum, this method also affects the secretion of cytokines. Thus, immortalized sebaceous gland cells cultured accordingly are considered as models inappropriately for investigating key characteristics of acne pathogenesis (Zouboulis CC, Hossini AM, Hou X, Wang C, Weylandt KH, Pietzner A. Effects of Moringa oleifera Seed Oil on Cultured Human Sebocytes In Vitro and Comparison with Other Oil Types. Int J Mol Sci. 2023 Jun 19; 24 (12): 10332). As described in non-patent document 3, when sebaceous gland cells are isolated from the human skin using a microsurgical instrument and primarily cultured, the accumulation of lipids can be observed in the cytoplasm, but these sebaceous gland cells can only grow for 3 to 6 generations. In addition, only a limited quantity of cells can be acquired, and the acquired cells cannot be efficiently expanded. This hinders the sustained, large-scale, and in-depth research. If sebaceous gland cells are isolated from multiple donors, these cells demonstrate heterogeneous characteristics, and it is challenging to avoid the instability in research outcomes, which undermines the comparability of findings across different studies (Xia LQ, Zouboulis C, Detmar M, et al. Isolation of human sebaceous glands and cultivation of sebaceous gland-derived cells as an in vitro model [J]. J Invest Dermatol, 1989, 93 (3): 315-321).

Therefore, there is an urgent need in the art for a method to prepare human sebaceous progenitor cells at a high purity and yield without using animal cells. This method is applicable for providing relevant *in vitro* models to enable disease modeling and develop innovative treatments.

### CONTENT OF THE INVENTION

An objective of the present disclosure is to construct a stable human *in vitro* sebaceous gland cell to address the issues mentioned in the background.

To achieve the above objective, the present disclosure provides an immortalized human sebaceous gland progenitor cell, a preparation method thereof, and a method for screening and evaluating oil-regulating drugs or skincare products using the same.

The present disclosure provides an immortalized human sebaceous gland progenitor cell. The immortalized human sebaceous gland progenitor cell was deposited at the China Center for Type Culture Collection (CCTCC) in Wuhan University, Wuhan, China on October 10, 2023, with an accession number of CCTCC NO: C2023286 and a classification designation of human induced pluripotent stem cell (hiPSC)-derived sebaceous gland progenitor cell iPS-SBP1#.

The present disclosure also provides a preparation method of the immortalized human sebaceous gland progenitor cell, including the following steps:
(1) isolation and expansion culture of a renal epithelial cell:
1) the isolation of the renal epithelial cell:
   a. collecting urine from an acne patient in a 50 mL centrifuge tube; and conducting centrifugation at 400 × g and room temperature for 10 min, and discarding a resulting supernatant with 1 mL to 2 mL of the supernatant left;
   b. resuspending a resulting pellet with 10 mL of a washing solution from a UrinEasy urine cell isolation and culture kit, and transferring to another centrifuge tube; conducting centrifugation at 200 × g and room temperature for 10 min, and discarding a resulting supernatant; and resuspending a resulting cell pellet with a medium from the UrinEasy urine cell isolation and culture kit, and inoculating into a culture plate coated with Matrigel in advance;
   c. culturing for 24 h, and subsequently, observing whether there is contamination; and if there is no contamination, adding 800 µL of the medium from the UrinEasy urine cell isolation and culture kit, and further culturing for 3 d; and
   d. on day 4, monitoring for contamination and adherent cells; if there is no contamination, discarding 2/3 of a medium in each well, and further adding 800 µL of the medium from the UrinEasy urine cell isolation and culture kit; and subsequently, monitoring daily for contamination and adherent cells, and conducting a partial-medium or complete-medium change with the medium every other day; and
2) the expansion culture of the renal epithelial cell:
   a. after the isolation in the step 1), when a confluency of the renal epithelial cell reaches 80% to 90%, allowing passage and expansion;
   b. discarding a medium in each well, and washing once with phosphate buffered saline (PBS); adding a trypsin digestion solution at a mass concentration of 0.25%, and conducting digestion in a 37°C incubator for 3 min; and adding an expansion culture basal medium from a UrinEasy urine cell expansion culture kit in a volume twice a volume of the trypsin digestion solution to terminate the digestion, and gently rinsing a bottom surface of each culture well to make cells detach; and
   c. collecting a resulting cell suspension in a 15 mL centrifuge tube; conducting centrifugation at 1,200 rpm and room temperature for 3 min, and discarding a resulting supernatant; resuspending a resulting cell pellet with the expansion culture basal medium from the UrinEasy urine cell expansion culture kit, and gently pipetting up and down to produce a single-cell suspension; and inoculating the single-cell suspension evenly into a culture plate coated with Matrigel;

(2) establishment and expansion culture of a hiPSC line:
1) the establishment of the hiPSC line:
   a. when the renal epithelial cell derived from the urine in the step (1) is passaged, inoculating the renal epithelial cell at cell densities of 5,000/well, 10,000/well, and 15,000/well into a 96-well plate coated with Matrigel, with three replicate wells for each density, wherein the day on which the step a is conducted is recorded as day -1;
   b. the next day, observing a cell status under a microscope, selecting a well from each density for cell counting, and selecting a culture well with a cell count of 10,000 to 20,000 for subsequent experiments;
   c. mixing 10 µL of a reprogramming additive I with Catalog No. CA5002002-1 and 10 mL of a medium from the UrinEasy urine cell expansion culture kit thoroughly to prepare a reprogramming medium A; mixing 100 µL of the reprogramming medium A and 10 µL of a reprogramming additive II with Catalog No. CA5002002-2 thoroughly to prepare a reprogramming medium B; and discarding a medium in the selected culture well, and adding the reprogramming medium B, wherein the day on which the step c is conducted is recorded as day 0;
   d. observing a cell morphological change daily under a microscope, and culturing consecutively for 2 d;
   e. on day 3, observing a cell morphology under a microscope; and if the cell morphology changes significantly and a confluency of cells reaches 100%, allowing passage;
   f. when the passage is conducted, adding 10 µL of a reprogramming additive III with Catalog No. CA5002002-3 to 10 mL of the medium from the UrinEasy urine cell expansion culture kit, and thoroughly mixing to prepare a reprogramming medium C; discarding a medium in a cell culture well to be digested, and washing once with PBS; adding 50 µL of trypsin to the cell culture well, and conducting digestion in a 37°C incubator for 3 min; adding 100 µL of the reprogramming medium C to terminate the digestion, and rinsing a bottom surface of the cell culture well; collecting a resulting cell suspension in a centrifuge tube with the reprogramming medium C, and pipetting up and down for thorough mixing; and inoculating suspended cells evenly into a culture plate coated with Matrigel at a passaging ratio of 1:6 to 1:10;
   g. on day 4, conducting a medium change with the medium from the UrinEasy urine cell expansion culture kit;
   h. monitoring a cell status daily, and if small cluster colonies are observed under a microscope, conducting a medium change daily using a Reproeasy human cell reprogramming basal medium with Catalog No. CA5003050-1;
   i. if a plurality of clones each composed of 10 or more cells are observed, replacing an original medium by a PGM1 human pluripotent stem cell (PSC) medium with Catalog No. CA1007500;
   j. observing cells under a microscope; when the plurality of clones grow to fill a field of view under a 10X objective lens, cutting the plurality of clones using a 1 mL syringe needle, and separating the plurality of clones from a bottom surface of a culture well using a 200 µL sterile pipette tip; and transferring the plurality of clones to a Matrigel-coated 24-well plate with the PGM1 human PSC medium, and culturing in a 37°C incubator with a CO₂ volume concentration of 5% and a humidity of 90% or more; and
   k. observing daily, and conducting a medium change with the PGM1 human PSC medium; and
2) the expansion culture of the hiPSC line:
   a. when it is observed under a microscope that cells grow to a confluency of about 80% and are in a prominent status, or when clones are unevenly distributed and individual clones are relatively large, allowing passage; and
   b. discarding a medium in a well, and washing once with PBS; adding a DissoEasy human PSC digestion solution with Catalog No. CA1023100, and conducting digestion at room temperature for 3 min to 5 min; removing the DissoEasy human PSC digestion solution in the well, and adding the PGM1 human PSC medium; gently rinsing a bottom surface of the well in a fan-shaped pattern to make cells detach, and collecting a resulting cell suspension in a centrifuge tube; inoculating the cell suspension into a Matrigel-coated culture plate at a volume ratio of 1:5 to 1:10, and culturing in a 37°C incubator with a CO₂ volume concentration of 5% and a humidity of 90% or more; and subsequently, observing daily, and conducting a medium change daily with the PGM1 human PSC medium; and

(3) differentiation of the sebaceous gland progenitor cell
a. preparing a basal differentiation medium for hiPSC obtained in the step (2) to differentiate into a sebaceous gland progenitor cell, and replacing an original medium for the hiPSC with the basal differentiation medium, where the basal differentiation medium includes the following components:

| | |
|---|---|
| Dulbecco's Modified Eagle Medium (DMEM) : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| retinoic acid (RA) | 1 µM; |

b. conducting a medium change daily with the basal differentiation medium prepared in the step (a), and culturing consecutively for 3 d in a 37°C incubator with a CO₂ volume concentration of 5%;
c. after the 3 d culturing, replacing the basal differentiation medium with a sebaceous gland progenitor cell differentiation activation medium, where the sebaceous gland progenitor cell differentiation activation medium is produced by adding an epidermal growth factor at 20 ng/mL and a bone morphogenetic protein 4 at 1 ng/mL to the basal differentiation medium in the step (a);
d. conducting a medium change with the sebaceous gland progenitor cell differentiation activation medium every other day, and culturing consecutively for 7 d to 8 d in a 37°C incubator with a CO₂ volume concentration of 5%;
e. preparing a sebaceous gland progenitor cell differentiation medium including the following components:
EpiLife^{™} medium : Dermacult keratinocyte expansion medium volume ratio: 1:1

| | |
|---|---|
| epidermal growth factor | 20 ng/mL and |
| bone morphogenetic protein 4 | 1 ng/mL; |

f. using the sebaceous gland progenitor cell differentiation medium, and culturing consecutively until day 10 to day 11 in a 37°C incubator with a CO₂ volume concentration of 5%; digesting cells with TrypLE in a CO₂ incubator at 37°C for 15 min; conducting centrifugation at 200 × g for 3 min, and discarding a resulting supernatant; and resuspending resulting cells in the sebaceous gland progenitor cell differentiation medium supplemented with a dihydrochloride ATP-competitive ROCK inhibitor at 10 µM;
g. inoculating the cells obtained in the step (f) into a culture plate coated with fibronectin at 25 µg/mL;
h. culturing consecutively until day 12 to day 13 in a 37°C incubator with a CO₂ volume concentration of 5%, and replacing an original medium with a fresh sebaceous gland progenitor cell differentiation medium; and
i. conducting a medium change daily, and culturing consecutively for 7 d to 9 d in a 37°C incubator with a CO₂ volume concentration of 5% to produce the sebaceous gland progenitor cell.

The present disclosure further provides a method for screening and evaluating oil-regulating drugs or skincare products using the progenitor cell, including: directly co-incubating the sebaceous gland progenitor cell with dihydrotestosterone (DHT) at room temperature for 48 h to stimulate the sebaceous gland progenitor cell to generate increased lipids, thereby enabling the screening and evaluating of the oil-regulating drugs or the skincare products; alternatively, culturing the sebaceous gland progenitor cell to differentiate into a mature sebaceous gland cell, and using the mature sebaceous gland cell to screen and evaluate the oil-regulating drugs or the skincare products.

Further, when the sebaceous gland progenitor cell is cultured to differentiate into a mature sebaceous gland cell and the mature sebaceous gland cell is then used to screen and evaluate the oil-regulating drugs or the skincare products, a method for inducing the sebaceous gland progenitor cell to differentiate into the mature sebaceous gland cell is as follows:
a. preparing a basal differentiation medium for the mature sebaceous gland cell, where the basal differentiation medium includes the following components:

| | |
|---|---|
| DMEM : Ham's F12 | volume ratio: 3:1 |
| fetal bovine serum | mass concentration: 2.5% |
| insulin | 10 ng/mL |
| epidermal growth factor | 3 ng/mL |
| hydrocortisone | 45.2 ng/mL |
| isoprenaline | 1 µM and |
| adenine | 24 µg/mL; |

b. subjecting the sebaceous gland progenitor cell to digestion for 15 min with TrypLE in a 37°C incubator with a CO₂ volume concentration of 5%; conducting centrifugation at 250 × g for 3 min, and discarding a resulting supernatant; adding a dihydrochloride ATP-competitive ROCK inhibitor at 10 µM to the basal differentiation medium for the mature sebaceous gland cell, and using a resulting medium to resuspend a resulting cell pellet; and inoculating into a culture plate with a cell density of 2.5 × 10⁴ cells/cm², and culturing overnight in a 37°C incubator with a CO₂ volume concentration of 5%;
c. the next day, conducting a medium change with the basal differentiation medium for the mature sebaceous gland cell, and adding a TGF-β/Smad inhibitor at 10 µM;
d. conducting a medium change daily, and culturing consecutively for 3 d in a 37°C incubator with a CO₂ volume concentration of 5%;
e. 3 d later, replacing the basal differentiation medium for the mature sebaceous gland cell with a final differentiation medium for the mature sebaceous gland cell, and culturing in a 37°C incubator with a CO₂ volume concentration of 5%; and
f. conducting a medium change daily, and culturing consecutively for 3 d to 6 d in a 37°C incubator with a CO₂ volume concentration of 5% to produce the mature sebaceous gland cell.

Further, the final differentiation medium for the mature sebaceous gland cell in the step (e) is prepared by adding a TGF-β/Smad inhibitor at 10 µM to the basal differentiation medium for the mature sebaceous gland cell; or is prepared by adding the TGF-β/Smad inhibitor at 10 µM and a PPAR-β/δ agonist at 1 µM to the basal differentiation medium for the mature sebaceous gland cell; or is prepared by adding the TGF-β/Smad inhibitor at 10 µM, a GW0742 PPAR-β/δ agonist at 1 µM, and cyclopamine at 5 µM to the basal differentiation medium for the mature sebaceous gland cell.

In summary, the present disclosure has the following beneficial effects: (1) In the preparation method of the immortalized human sebaceous gland progenitor cell provided in the present disclosure, hiPSCs derived from urine of an acne patient are adopted for culture and differentiation. The acne patient inherently exhibits excessive sebum secretion. Thus, the preparation method provides a foundational guarantee for enabling the effective lipid secretion *in vitro* subsequently. (2) In the preparation method of the immortalized human sebaceous gland progenitor cell provided in the present disclosure, when the hiPSCs differentiate into sebaceous gland progenitor cells, the sebaceous gland progenitor cells acquired undergo high-level expression of markers for sebaceous gland progenitor cells, and there is stable and accurate differentiation. (3) In the immortalized human sebaceous gland progenitor cell prepared in the present disclosure, the expression of genes related to liquid droplet formation, lipid storage, and AR is significantly enhanced. Moreover, the immortalized human sebaceous gland progenitor cell exhibits high stability. As a result, the immortalized human sebaceous gland progenitor cell can be well used for the screening and evaluation of oil-regulating drugs or skincare products. (4) When the progenitor cell in the present disclosure is used for screening and evaluating oil-regulating drugs or skincare products, the progenitor cell can be further cultured to differentiate into a mature sebaceous gland cell. The mature sebaceous gland cell produced can highly express the markers, and possesses the ability of producing and accumulating liquid droplets like sebaceous gland cells in the human. The androgen stimulation can accelerate the maturation and differentiation of progenitor cells to release increased liquid droplets. The mature sebaceous gland cell has well-defined genetic information and clinical background, is stable, and can simulate behaviors of sebaceous glands in acne patients *in vitro.* Therefore, the mature sebaceous gland cell can be accurately and conveniently used to screen and evaluate oil-regulating drugs or skincare products.

### DESCRIPTION OF THE DRAWINGS

FIG. 1 shows morphologies of renal epithelial cells isolated from a urine sample at different time points of culturing in the present disclosure;
FIG. 2 shows optical microscopy images of hiPSCs at different time points after a transcription factor is added in the present disclosure;
FIG. 3 shows a morphology of hiPSCs produced by reprogramming renal epithelial cells after expansion culture in the present disclosure;
FIG. 4 shows alkaline phosphatase staining results of hiPSCs in the present disclosure;
FIG. 5 shows immunofluorescence staining results of hiPSCs in the present disclosure;
FIG. 6 shows karyotype analysis results for hiPSCs in the present disclosure;
FIG. 7 shows staining results for teratoma formation and trilineage differentiation of hiPSCs in the present disclosure;
FIG. 8 shows morphological changes of sebaceous gland progenitor cells during differentiation in the present disclosure;
FIG. 9 is a comparison diagram of expression levels of sebaceous gland progenitor cell marker genes between a human keratinocyte (HaCaT) and the sebaceous gland progenitor cell (SBP) of the present disclosure, where **P <* 0.05, ***P* < 0.01, and ****P* < 0.001 vs HaCaT;
FIG. 10 is a comparison diagram of expression levels of sebaceous gland progenitor cell marker genes between an immortalized human sebaceous gland cell (SZ95) and the sebaceous gland progenitor cell (SBP) of the present disclosure, where ^{#}*P* < 0.05, *^{##}P* < 0.01, and ^{###}*P* < 0.001 vs SZ95;
FIG. 11 is a comparison diagram of expression levels of sebaceous gland progenitor cell marker genes between hiPSC and the sebaceous gland progenitor cell (SBP) of the present disclosure, where *^{&}P <* 0.05, *^{&&}P* < 0.01, and *^{&&&}P* < 0.001 vs hiPSC;
FIG. 12 shows comparative images of oil red O staining results for detecting liquid droplet contents in a human keratinocyte (HaCaT), an immortalized human sebaceous gland cell (SZ95), hiPSC, and the sebaceous gland progenitor cell (SBP) of the present disclosure;
FIG. 13 is a comparative statistical graph of oil red O staining results for detecting liquid droplet contents in a human keratinocyte (HaCaT), an immortalized human sebaceous gland cell (SZ95), hiPSC, and the sebaceous gland progenitor cell (SBP) of the present disclosure;
FIG. 14 shows comparative images of Nile red fluorescence staining results for detecting intracellular liquid droplet fluorescence intensities in a human keratinocyte (HaCaT), an immortalized human sebaceous gland cell (SZ95), hiPSC, and the sebaceous gland progenitor cell (SBP) of the present disclosure;
FIG. 15 shows RNA-seq analysis results of gene expression differences between an immortalized human sebaceous gland cell (SZ95) and the sebaceous gland progenitor cell (SBP) of the present disclosure;
FIG. 16 shows liquid droplet changes detected by oil red O staining after cells are treated with DHT at different concentrations;
FIG. 17 shows liquid droplet changes detected by oil red O staining after sebaceous gland progenitor cells (SBP) are treated with drug and skincare ingredients at different concentrations;
FIG. 18 shows morphological observation images of mature sebaceous gland cells (SBM) obtained in Examples 3 to 5 of the present disclosure;
FIG. 19 shows liquid droplet formation and cytokeratin expression in mature sebaceous gland cells (SBM) of Example 3 of the present disclosure, where arrows indicate liquid droplet and KRT7 expression;
FIG. 20 shows responses of the mature sebaceous gland cells (SBM) obtained in Examples 3 to 5 of the present disclosure to DHT;
FIG. 21 shows BODIP staining results of liquid droplets of the mature sebaceous gland cells (SBM) obtained in Examples 3 to 5 of the present disclosure, where arrows indicate liquid droplet expression; and
FIG. 22 shows oil red O staining results for morphological changes of mature sebaceous gland cells (SBM) treated with drug and skincare ingredients at different concentrations, where a boxed content shows a morphology of a mature sebaceous gland cell (SBM) with a volume increasing after a DHT intervention.

### SPECIFIC IMPLEMENTATIONS

The specific embodiments of the present disclosure are described in detail below. It should be understood that the specific embodiments described here are only intended to illustrate and explain the present disclosure, and are not intended to limit the present disclosure. Unless otherwise specified, experiments adopted below are conducted at room temperature, and reagents adopted below have the following supplier information: A UrinEasy urine cell isolation and culture kit, a reprogramming additive I , a reprogramming additive II, a reprogramming additive III, a Reproeasy human cell reprogramming basal medium, a PGM1 human PSC medium, and a DissoEasy human PSC digestion solution are purchased from Beijing Cellapy Biotechnology Co., Ltd. A EpiLife^{™} medium with Catalog No. MEPI500CA is purchased from Thermo Fisher Scientific. A Dermacult keratinocyte expansion medium is purchased from Jiangsu Real-gen Biotechnology Co., Ltd.

Example 1: The present disclosure provides an immortalized human sebaceous gland progenitor cell deposited at the China Center for Type Culture Collection (CCTCC) under an accession number of CCTCC NO: C2023286. A preparation method of the immortalized human sebaceous gland progenitor cell includes the following steps:
(1) Isolation and expansion culture of a renal epithelial cell:
1) The isolation of the renal epithelial cell:
   a. Urine was collected from an acne patient in a 50 mL centrifuge tube and centrifuged at 400 × g and room temperature for 10 min. A resulting supernatant was discarded with 1 mL of the supernatant left.
   b. A resulting pellet was resuspended with 10 mL of a washing solution from a UrinEasy urine cell isolation and culture kit, transferred to another centrifuge tube, and centrifuged at 200 × g and room temperature for 10 min. A resulting supernatant was discarded. A resulting cell pellet was resuspended with a medium from the UrinEasy urine cell isolation and culture kit, and inoculated into a culture plate coated with Matrigel in advance.
   c. Culturing was conducted for 24 h, and subsequently, whether there was contamination was observed. If there was no contamination, 800 µL of the medium from the UrinEasy urine cell isolation and culture kit was added, and culturing was further conducted for 3 d.
   d. On day 4, monitoring was conducted for contamination and adherent cells. If there was no contamination, 2/3 of a medium in each well was discarded, and 800 µL of the medium from the UrinEasy urine cell isolation and culture kit was further added. Subsequently, monitoring was conducted daily for contamination and adherent cells, and a partial-medium or complete-medium change was conducted with the medium every other day.
2) The expansion culture of the renal epithelial cell:
   a. After the isolation in the step 1), when a confluency of the renal epithelial cell reached 80%, passage and expansion were allowed.
   b. A medium in each well was discarded, and washing was conducted once with PBS. A trypsin digestion solution at a mass concentration of 0.25% was added, and digestion was conducted in a 37°C incubator for 3 min. An expansion culture basal medium from a UrinEasy urine cell expansion culture kit was added in a volume twice a volume of the trypsin digestion solution to terminate the digestion, and a bottom surface of each culture well was gently rinsed to make cells detach.
   c. A resulting cell suspension was collected in a 15 mL centrifuge tube and centrifuged at 1,200 rpm and room temperature for 3 min. A resulting supernatant was discarded. A resulting cell pellet was resuspended with the expansion culture basal medium from the UrinEasy urine cell expansion culture kit, and gently pipetted up and down to produce a single-cell suspension. The single-cell suspension was inoculated evenly into a culture plate coated with Matrigel.

(2) Establishment and expansion culture of a hiPSC line:
1) The establishment of the hiPSC line:
   a. When the renal epithelial cell derived from the urine in the step (1) was passaged, the renal epithelial cell was inoculated at cell densities of 5,000/well, 10,000/well, and 15,000/well into a 96-well plate coated with Matrigel, with three replicate wells for each density. The day on which the step a was conducted was recorded as day -1.
   b. The next day, a cell status was observed under a microscope, and a well was selected from each density for cell counting. A culture well with a cell count of 10,000 to 20,000 was selected for subsequent experiments.
   c. 10 µL of a reprogramming additive I with Catalog No. CA5002002-1 and 10 mL of a medium from the UrinEasy urine cell expansion culture kit were mixed thoroughly to prepare a reprogramming medium A. 100 µL of the reprogramming medium A and 10 µL of a reprogramming additive II with Catalog No. CA5002002-2 were mixed thoroughly to prepare a reprogramming medium B. A medium in the selected culture well was discarded, and the reprogramming medium B was added. The day on which the step c was conducted was recorded as day 0.
   d. A cell morphological change was observed daily under a microscope, and culturing was conducted consecutively for 2 d.
   e. On day 3, a cell morphology was observed under a microscope. If the cell morphology changed significantly and a confluency of cells reached 100%, passage was allowed.
   f. When the passage was conducted, 10 µL of a reprogramming additive III with Catalog No. CA5002002-3 was added to 10 mL of the medium from the UrinEasy urine cell expansion culture kit, and thorough mixing was conducted to prepare a reprogramming medium C. A medium in a cell culture well to be digested was discarded, and washing was conducted once with PBS. 50 µL of trypsin was added to the cell culture well, and digestion was conducted in a 37°C incubator for 3 min. 100 µL of the reprogramming medium C was added to terminate the digestion, and a bottom surface of the cell culture well was rinsed. A resulting cell suspension was collected in a centrifuge tube with the reprogramming medium C, and pipetted up and down for thorough mixing. Suspended cells were inoculated evenly into a culture plate coated with Matrigel at a passaging ratio of 1:6.
   g. On day 4, a medium change was conducted with the medium from the UrinEasy urine cell expansion culture kit.
   h. A cell status was monitored daily. If small cluster colonies were observed under a microscope, a medium change was conducted daily using a Reproeasy human cell reprogramming basal medium with Catalog No. CA5003050-1.
   i. If a plurality of clones each composed of 10 or more cells were observed, an original medium was replaced by a PGM1 human PSC medium with Catalog No. CA1007500.
   j. Cells were observed under a microscope. When the plurality of clones grew to fill a field of view under a 10X objective lens, the plurality of clones were cut using a 1 mL syringe needle, and separated from a bottom surface of a culture well using a 200 µL sterile pipette tip. Then, the plurality of clones were transferred to a Matrigel-coated 24-well plate with the PGM1 human PSC medium, and cultured in a 37°C incubator with a CO₂ volume concentration of 5% and a humidity of 90% or more.
   k. Observation was conducted daily, and a medium change was conducted with the PGM1 human PSC medium.
2) The expansion culture of the hiPSC line:
   a. When it was observed under a microscope that cells grew to a confluency of about 80% and were in a prominent status, or when clones were unevenly distributed and individual clones were relatively large, passage was allowed.
   b. A medium in a well was discarded, and washing was conducted once with PBS. A DissoEasy human PSC digestion solution with Catalog No. CA1023100 was added, and digestion was conducted at room temperature for 3 min. The DissoEasy human PSC digestion solution in the well was removed, and the PGM1 human PSC medium was added. A bottom surface of the well was gently rinsed in a fan-shaped pattern to make cells detach, and a resulting cell suspension was collected in a centrifuge tube. The cell suspension was inoculated into a Matrigel-coated culture plate at a volume ratio of 1:5, and cultured in a 37°C incubator with a CO₂ volume concentration of 5% and a humidity of 90% or more. Subsequently, observation was conducted daily, and a medium change was conducted daily with the PGM1 human PSC medium.

(3) Differentiation of the sebaceous gland progenitor cell
a. A basal differentiation medium for hiPSC obtained in the step (2) to differentiate into a sebaceous gland progenitor cell was prepared. An original medium for the hiPSC was replaced with the basal differentiation medium. The basal differentiation medium included the following components:

| | |
|---|---|
| DMEM : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| RA | 1 µM. |

b. A medium change was conducted daily with the basal differentiation medium prepared in the step (a), and culturing was conducted consecutively for 3 d in a 37°C incubator with a CO₂ volume concentration of 5%.
c. After the 3 d culturing, the basal differentiation medium was replaced with a sebaceous gland progenitor cell differentiation activation medium. The sebaceous gland progenitor cell differentiation activation medium was produced by adding an epidermal growth factor at 20 ng/mL and a bone morphogenetic protein 4 at 1 ng/mL to the basal differentiation medium in the step (a).
d. A medium change was conducted with the sebaceous gland progenitor cell differentiation activation medium every other day, and culturing was conducted consecutively for 7 d in a 37°C incubator with a CO₂ volume concentration of 5%.
e. A sebaceous gland progenitor cell differentiation medium was prepared. The sebaceous gland progenitor cell differentiation medium included the following components:
EpiLife^{™} medium : Dermacult keratinocyte expansion medium volume ratio: 1:1

| | |
|---|---|
| epidermal growth factor | 20 ng/mL and |
| bone morphogenetic protein 4 | 1 ng/mL. |

f. The sebaceous gland progenitor cell differentiation activation medium was replaced with the sebaceous gland progenitor cell differentiation medium, and culturing was conducted consecutively for 10 d in a 37°C incubator with a CO₂ volume concentration of 5%. Cells were digested with TrypLE in a CO₂ incubator at 37°C for 15 min. Centrifugation was conducted at 200 × g for 3 min, and a resulting supernatant was discarded. Resulting cells were resuspended in the sebaceous gland progenitor cell differentiation medium supplemented with a dihydrochloride ATP-competitive ROCK inhibitor at 10 µM.
g. The cells obtained in the step (f) were inoculated into a culture plate coated with fibronectin at 25 µg/mL.
h. Culturing was conducted consecutively until day 12 in a 37°C incubator with a CO₂ volume concentration of 5%, and an original medium was replaced with a fresh sebaceous gland progenitor cell differentiation medium.
i. A medium change was conducted daily, and culturing was conducted consecutively for 7 d in a 37°C incubator with a CO₂ volume concentration of 5% to produce the sebaceous gland progenitor cell.

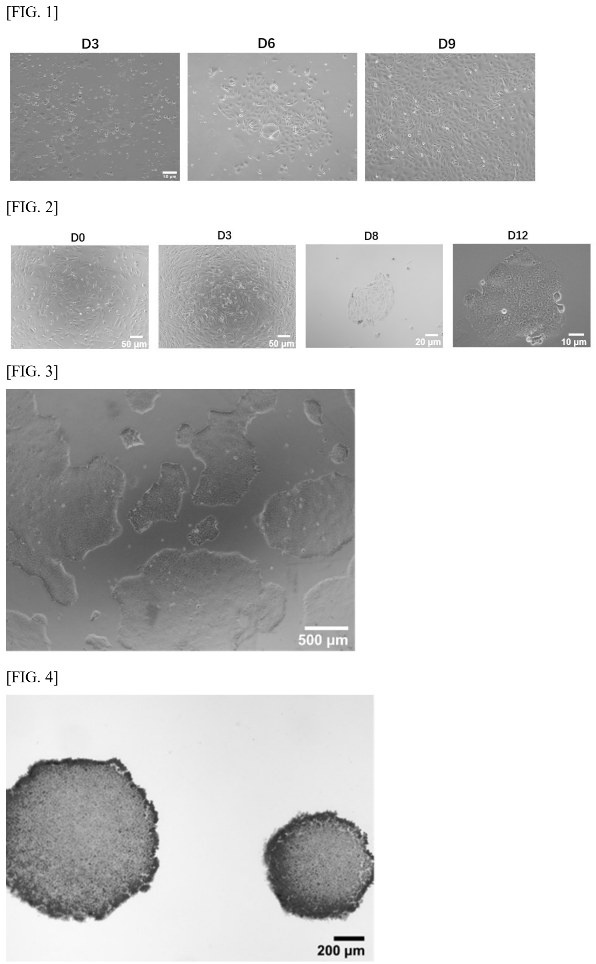

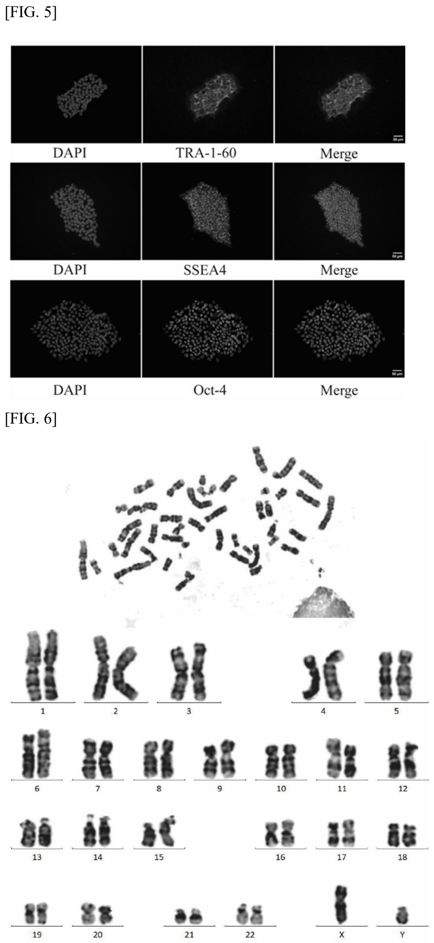

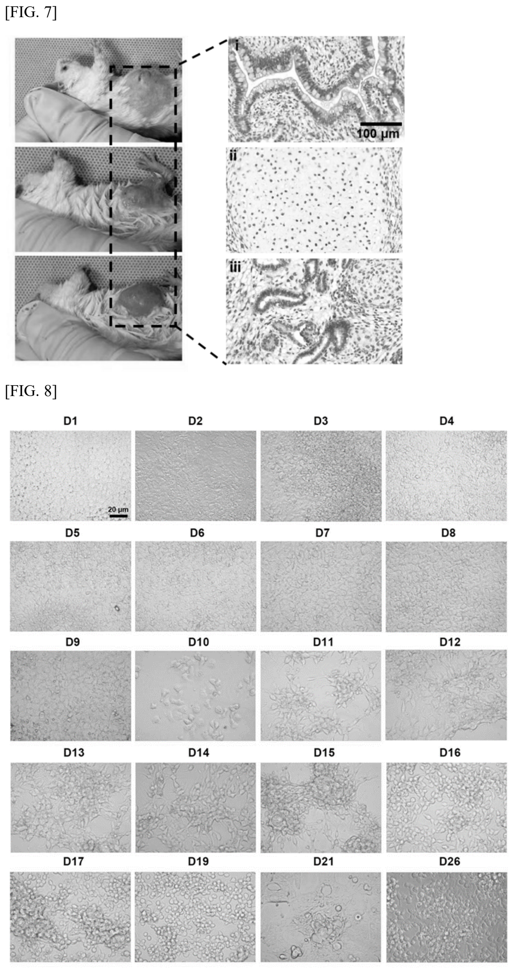

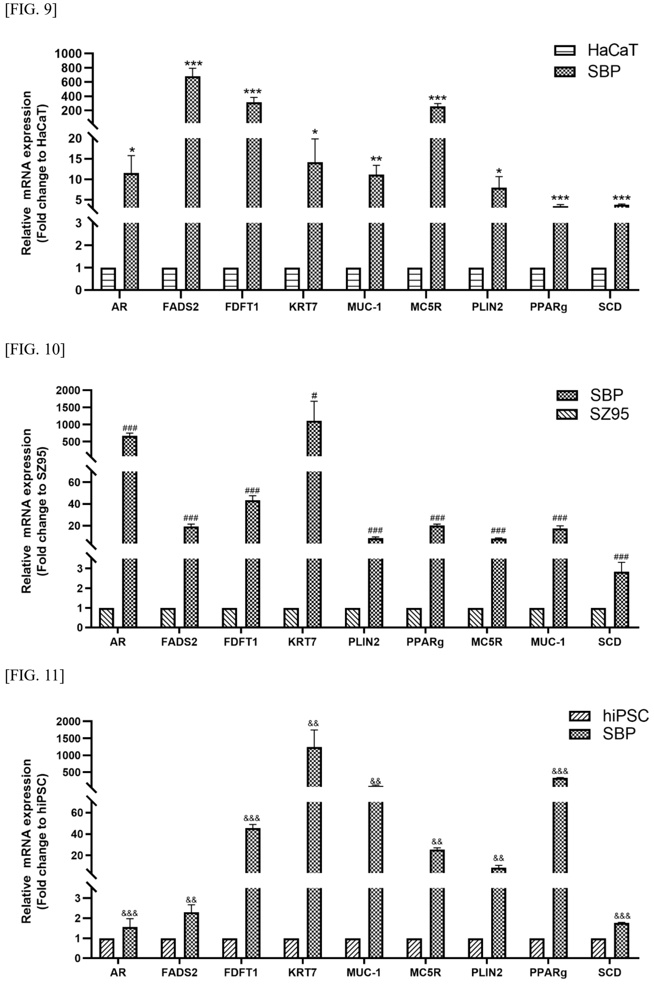

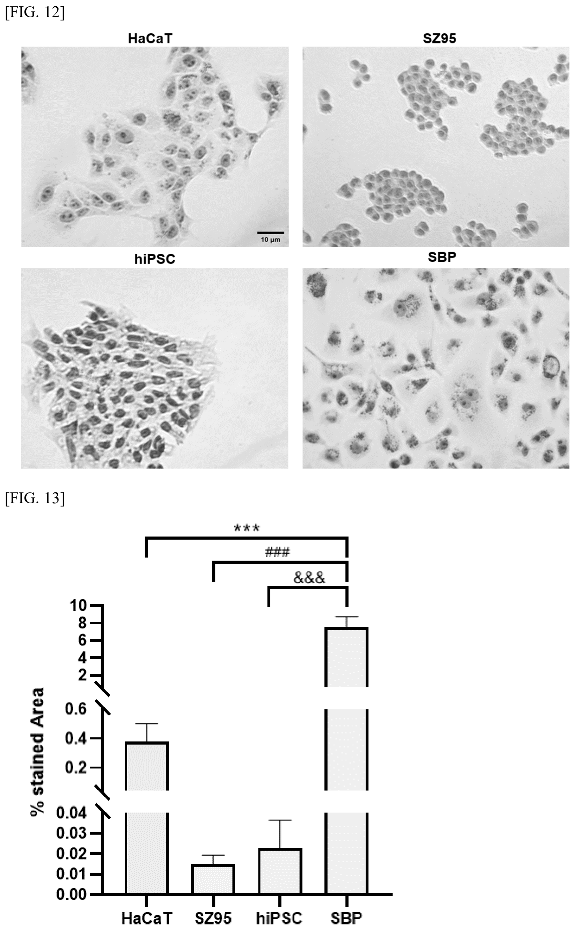

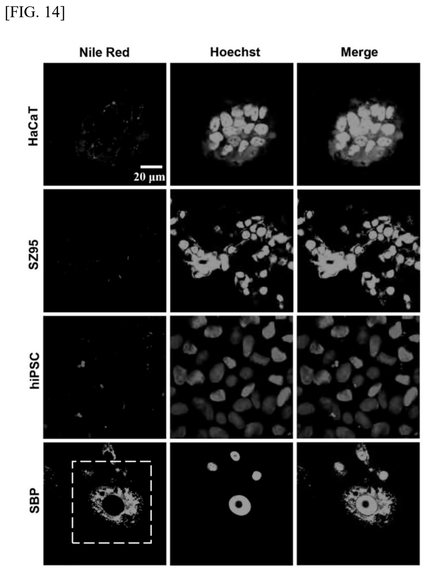

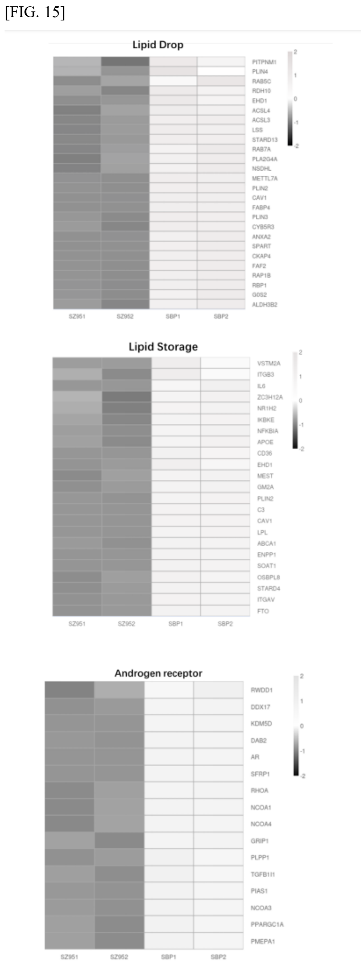

Through the following experiments, it has been proved that the sebaceous gland progenitor cell obtained in the present disclosure can stably proliferate, exhibits excellent liquid droplet formation and lipid storage abilities, and is well-suited for the screening and evaluation of oil-regulating drugs or skincare products.
**1.** 1. Isolation and expansion of renal epithelial cells
   1) 1) Isolation and culture of renal epithelial cells
      **a.** Cells were isolated from a urine sample and cultured to produce renal epithelial cells. At different time points (day 3, day 6, and day 9) of the culturing, a cell morphology was observed and imaged under an optical microscope.
      **b.** As shown in FIG. 1, with the passage of time and the constant medium changes, squamous epithelial cells growing in a suspended state and impurities were gradually reduced, and a plurality of clusters of typical adherent cells with a spindle-like or cobblestone-like morphology could be observed under the optical microscope, indicating the successful isolation of renal epithelial cells.
**2.** Establishment, culture, and identification of hiPSCs
   1) Establishment of hiPSCs
      **a.** A transcription factor was added. At different time points (day 0, day 3, day 8, and day 12) after the transcription factor was added, a cell morphology was observed and imaged under an optical microscope.
      **b.** As shown in FIG. 2, in contrast to day 0, on day 3 after the transcription factor was added, the cell morphology significantly changed. A cell shape became irregular, and partial cells were dead and detached. After cells were continuously cultured until day 12, the growth of typical clones could be observed. Round-like cells in each clone were tightly connected and had a high nucleusto-cytoplasm ratio, and these clones had clear and sharp edges, which were consistent with the morphological characteristics of hiPSCs.
   2) Culture of hiPSCs
      **a.** Cells were subjected to mechanical isolation and expansion. Then, a cell morphology was observed and imaged under an optical microscope.
      **b**. As shown in FIG. 3, clones presented a typical morphology with a sharp edge, and no significantly-differentiated cells were observed. Cells in a clone were tightly connected and had large nuclei and prominent states. Thus, these cells could be used for subsequent identification and differentiation experiments.
   3) Identification of pluripotency of hiPSCs by alkaline phosphatase staining
      **a**. hiPSCs were inoculated evenly into a 24-well plate, and cultured. When resulting clones had an appropriate size without excessive confluence, the corresponding experiment could be conducted. Staining was conducted using an alkaline phosphatase staining kit. A staining solution should be prepared in the dark. Specifically, a solution A and a solution B were thoroughly mixed in a volume ratio of 1:1, and then used within 30 min. A medium in a well was removed, cells were washed once with PBS, and then the PBS was removed. A fixation solution was added at 500 µL/well, and fixation was conducted at room temperature for 5 min. Washing was conducted twice with PBS. Then, the staining solution was added at 500 µL/well, and incubation was conducted for 20 min at room temperature in the dark. After the incubation was completed, the staining solution in each well was discarded, and washing was conducted once with PBS. Then, PBS was added for storage to prevent cells from drying. Staining results were observed under a microscope.
      **b.** As shown in FIG. 4, clones in each field of view were stained dark-blue. The darker the blue, the higher the expression level of alkaline phosphatase. It could be seen that alkaline phosphatase was highly expressed in cells within the clones.
   4) Identification of pluripotency of hiPSCs by immunofluorescence staining
      **a.** hiPSCs were inoculated evenly into a 24-well plate, and cultured. When resulting clones had an appropriate size without excessive confluence, the corresponding experiment could be conducted. The plate was taken out from an incubator, a medium in each well was removed, and cells were washed once with PBS. 4% paraformaldehyde was added to cover a bottom surface of each well, and fixation was conducted at room temperature for 10 min. The fixation solution was discarded, and washing was conducted twice with PBS. Then, 500 µL of 0.2% Triton X-100 was added, and incubation was conducted at room temperature for 15 min. A liquid in each well was discarded. 500 µL of 1% bovine serum albumin (BSA) was added to cover a bottom surface, and blocking was conducted at room temperature for 1 h. The BSA was discarded. A primary antibody solution diluted appropriately was added, and incubation was conducted overnight at 4°C. The next day, the culture plate was taken out, a liquid in each well was discarded, and washing was conducted three times with PBS for 5 min each time. A secondary antibody solution diluted at 1:1,000 was added, and incubation was conducted at room temperature for 1 h. The secondary antibody solution was removed, and washing was conducted three times with PBS for 5 min each time. A 4',6-diamidino-2-phenylindole (DAPI) solution was added, and incubation was conducted at room temperature for 10 min. The DAPI solution was removed, and washing was conducted three times with PBS. PBS was added to cover a bottom surface to prevent cells from drying. In this case, observation could be directly conducted under an inverted fluorescence microscope.
      **b.** As shown in FIG. 5, three protein markers, TRA-1-60, SSEA4, and Oct-4, were selected for immunofluorescence staining in this experiment. hiPSCs resulting from the reprogramming of renal epithelial cells expressed all of the three protein markers.
   5) Identification of pluripotency of hiPSCs by karyotype analysis
      **a.** Conventionally, when cells were cultured to a confluency of approximately 80%, the experiment could be started. A PSC medium including colchicine at 0.1 µg/mL was added, and culturing was further conducted in an incubator for 1.5 h. The subsequent experiment was then started. Cells were digested with trypsin, and collected in a centrifuge tube. Centrifugation was conducted for 10 min at 1,000 rpm and room temperature, and a resulting supernatant was discarded. A 0.075 mol/L KCl solution was added, and a hypotonic treatment was conducted in a 37°C water bath for 20 min. 1 mL of a pre-fixation solution (including methanol and glacial acetic acid in a volume ratio of 3:1) was added, and thorough mixing was conducted. Centrifugation was conducted for 10 min at 1,000 rpm and room temperature. A resulting supernatant was discarded, and a resulting precipitate was retained. 8 mL of a fixation solution was added, and fixation was conducted for 20 min. Centrifugation was then conducted for 10 min at 1,000 rpm and room temperature. A resulting supernatant was discarded, and the fixation solution was added at an amount based on an amount of a resulting cell pellet. A wall of the centrifuge tube was tapped with fingers to resuspend cells. A glass slide was taken out from ice water. A resulting cell suspension was aspirated by a rubber tip dropper and dropped on the glass slide from a height of approximately 30 cm. The glass slide was dried at 80°C for 2 h. Trypsin was diluted with PBS to a final concentration of 0.25‰. The glass slide dried at 80°C for 2 h was treated in the diluted trypsin for 2 min to 3 min. A digestion time was determined based on a dryness degree of the glass slide. The glass slide was rinsed with PBS, then stained for 5 min to 10 min in a Giemsa stain, rinsed with tap water, dried at room temperature, mounted, and stored. Counting was conducted and chromosomal structural changes were analyzed under a microscope (FIG. 6).
      **b.** As shown in FIG. 6, G-banding analysis was conducted after cell culture. There were 46 chromosomes, which was normal. A sex chromosome consisted of XY, indicating a male karyotype. No abnormalities were observed in chromosomal structures.
   6) Identification of pluripotency of hiPSCs by a teratoma formation experiment
      **a.** After a sufficient quantity of cells were cultured according to experimental requirements, cells were digested and collected through centrifugation. Outer thigh regions of right hind legs of NOD-SCID mice were each injected with 0.3 mL of a hiPSC suspension using a syringe. The mice were subsequently raised under normal conditions. Tumor formation at an injection site was visible approximately 8 weeks to 12 weeks later. Photographic documentation was conducted. The mice were then sacrificed through cervical dislocation. A tumor formation site was incised using scissors to expose a visual field, and a tumor was carefully dissected using forceps and scissors. The tumor was fixed in a 10% formalin fixation solution for 24 h. A volume ratio of the fixation solution to the tumor was approximately 10:1. The tumor was then embedded and subjected to Hematoxylin and Eosin (H&E) staining. Staining results and tissue morphologies were observed using a slide scanner.
      **b**. As shown in FIG. 7, after 8 weeks to 12 weeks of differentiation, a protrusion was observed at an injection site, and a dissection result revealed subcutaneous tumor formation. Pathological H&E staining results of tumors collected revealed an endoderm-derived intestinal columnar epithelial tissue, a mesoderm-derived cartilage-like tissue, and an ectoderm-derived rosette-like neural tissue. This experiment showed that hiPSCs possessed the ability to differentiate into cells and tissues of the three germ layers *in vivo.*
**3.** Induced differentiation and lipogenic capacity evaluation of sebaceous gland progenitor cells
   1) Differentiation of sebaceous gland progenitor cells
      **a.** From day 1 to day 26 of the differentiation of hiPSCs into sebaceous gland progenitor cells, a cell morphology was observed and photographed under an optical microscope (scale = 20 µm).
      **b.** As shown in FIG. 8, in contrast to a morphology of hiPSCs, differentiating cells became spindle-shaped over time, resembling skin epidermal cells. At a later stage of differentiation, an increased cell volume and a distinct vacuole-like structure could be observed.
   2) Gene expression levels of markers for sebaceous gland progenitor cells
      **a.** Total RNA was extracted from cells using an RNA extraction kit from TransGen, and the extracted total RNA was reverse-transcribed into cDNA using a reverse transcription kit from TransGen. Gene expression levels of the following markers for sebaceous gland progenitor cells in cells were detected by reverse transcription-quantitative polymerase chain reaction (RT-qPCR): AR, fatty acid desaturase 2 (FADS2), farnesyl-diphosphate farnesyltransferase 1 (FDFT1), cytokeratin 7 (KRT7), perilipin 2 (PLIN2), peroxisome proliferator-activated receptor gamma (PPARG), type I transmembrane mucin (MUC1), melanocortin 5 receptor (MC5R), and stearoyl-CoA desaturase (SCD).
      **b.** As shown in FIG. 9 to FIG. 11, compared to a human keratinocyte (HaCaT), an immortalized human sebaceous gland cell (SZ95), and hiPSC, the sebaceous gland progenitor cell (SBP) obtained in the present disclosure expressed the markers for sebaceous gland progenitor cells at high levels.
   3) Detection of a liquid droplet content in sebaceous gland progenitor cells by oil red O staining
      **a.** Cells were inoculated into a 24-well plate and cultured. When a cell confluency reached 50%, staining was allowed. Cells were washed twice with PBS and fixed with 4% paraformaldehyde for 20 min. The fixation solution was removed, and washing was conducted twice with distilled water. A 60% isopropanol solution was added to immerse cells for 15 min, and then discarded. Washing was conducted twice with distilled water. A prepared oil red O staining solution was added, and staining was conducted for 20 min. The oil red O staining solution was removed, and washing was conducted with water until no staining solution remained. Nuclei were counterstained with a hematoxylin staining solution for 2 min. The hematoxylin staining solution was discarded, and washing was conducted with water until no staining solution remained. Distilled water was added to cover cells. The cells were observed and photographed under a microscope.
      **b**. As shown in FIG. 12 and FIG. 13, compared to a human keratinocyte (HaCaT), an immortalized human sebaceous gland cell (SZ95), and hiPSC, the sebaceous gland progenitor cell (SBP) obtained in the present disclosure produced abundant red liquid droplets according to oil red O staining results (scale = 10 µm).
   4) Detection of a liquid droplet fluorescence intensity in sebaceous gland progenitor cells by Nile red fluorescence staining
      **a.** Cells were inoculated into a chambered coverslip. When a cell confluency reached 50%, staining was allowed. Cells were washed twice with PBS, and fixed with 4% paraformaldehyde for 10 min. The fixation solution was removed, and washing was conducted twice with PBS. 1 mL of a Nile Red and Hoechst solution was added to each well, and incubation was conducted for 20 min at room temperature in the dark. Nile Red and Hoechst fluorescence images were acquired under a confocal laser scanning microscope. Different fluorescence staining images were merged. A boxed region indicated a liquid droplet expression region.
      **b.** As shown in FIG. 14, compared to a human keratinocyte (HaCaT), an immortalized human sebaceous gland cell (SZ95), and hiPSC, the sebaceous gland progenitor cell (SBP) obtained in the present disclosure had a high red fluorescence intensity according to Nile red fluorescence staining results, indicating a high liquid droplet content (scale = 20 µm).
   5) RNA-seq analysis of gene expression differences between sebaceous gland progenitor cells and immortalized human sebaceous gland cells
      **a.** Cellular RNA was extracted by a TRIzol method. The subsequent transcriptome sequencing was conducted by Beijing Novogene.
      **b.** According to sequencing results shown in FIG. 15, compared to the immortalized human sebaceous gland cell (SZ95), the sebaceous gland progenitor cell (SBP) obtained in the present disclosure demonstrated significantly-enhanced expression levels of genes related to liquid droplet formation, lipid storage, and AR.

It should be noted that all the aforementioned experimental results are average results of several parallel experiments. It can be seen that the sebaceous gland progenitor cell prepared by the preparation method of the present disclosure exhibits stable properties. The sebaceous gland progenitor cell can express markers for sebaceous gland progenitor cells at high levels, and demonstrates significantly-enhanced expression levels of genes related to liquid droplet formation, lipid storage, and AR. Thus, the sebaceous gland progenitor cell is highly suitable for the screening and evaluation of oil-regulating drugs or skincare products.

Example 2: The present disclosure provides an immortalized human sebaceous gland progenitor cell deposited at the China Center for Type Culture Collection (CCTCC) under an accession number of CCTCC NO: C2023286. A preparation method of the immortalized human sebaceous gland progenitor cell includes the following steps:
(1) Isolation and expansion culture of a renal epithelial cell:
1) The isolation of the renal epithelial cell:
   a. Urine was collected from an acne patient in a 50 mL centrifuge tube and centrifuged at 400 × g and room temperature for 10 min. A resulting supernatant was discarded with 2 mL of the supernatant left.
   b. A resulting pellet was resuspended with 10 mL of a washing solution from a UrinEasy urine cell isolation and culture kit, transferred to another centrifuge tube, and centrifuged at 200 × g and room temperature for 10 min. A resulting supernatant was discarded. A resulting cell pellet was resuspended with a medium from the UrinEasy urine cell isolation and culture kit, and inoculated into a culture plate coated with Matrigel in advance.
   c. Culturing was conducted for 24 h, and subsequently, whether there was contamination was observed. If there was no contamination, 800 µL of the medium from the UrinEasy urine cell isolation and culture kit was added, and culturing was further conducted for 3 d.
   d. On day 4, monitoring was conducted for contamination and adherent cells. If there was no contamination, 2/3 of a medium in each well was discarded, and 800 µL of the medium from the UrinEasy urine cell isolation and culture kit was further added. Subsequently, monitoring was conducted daily for contamination and adherent cells, and a partial-medium or complete-medium change was conducted with the medium every other day.
2) The expansion culture of the renal epithelial cell:
   a. After the isolation in the step 1), when a confluency of the renal epithelial cell reached 90%, passage and expansion were allowed.
   b. A medium in each well was discarded, and washing was conducted once with PBS. A trypsin digestion solution at a mass concentration of 0.25% was added, and digestion was conducted in a 37°C incubator for 3 min. An expansion culture basal medium from a UrinEasy urine cell expansion culture kit was added in a volume twice a volume of the trypsin digestion solution to terminate the digestion, and a bottom surface of each culture well was gently rinsed to make cells detach.
   c. A resulting cell suspension was collected in a 15 mL centrifuge tube and centrifuged at 1,200 rpm and room temperature for 3 min. A resulting supernatant was discarded. A resulting cell pellet was resuspended with the expansion culture basal medium from the UrinEasy urine cell expansion culture kit, and gently pipetted up and down to produce a single-cell suspension. The single-cell suspension was inoculated evenly into a culture plate coated with Matrigel.

(2) Establishment and expansion culture of a hiPSC line:
1) The establishment of the hiPSC line:
   a. When the renal epithelial cell derived from the urine in the step (1) was passaged, the renal epithelial cell was inoculated at cell densities of 5,000/well, 10,000/well, and 15,000/well into a 96-well plate coated with Matrigel, with three replicate wells for each density. The day on which the step a was conducted was recorded as day -1.
   b. The next day, a cell status was observed under a microscope, and a well was selected from each density for cell counting. A culture well with a cell count of 10,000 to 20,000 was selected for subsequent experiments.
   c. 10 µL of a reprogramming additive I with Catalog No. CA5002002-1 and 10 mL of a medium from the UrinEasy urine cell expansion culture kit were mixed thoroughly to prepare a reprogramming medium A. 100 µL of the reprogramming medium A and 10 µL of a reprogramming additive II with Catalog No. CA5002002-2 were mixed thoroughly to prepare a reprogramming medium B. A medium in the selected culture well was discarded, and the reprogramming medium B was added. The day on which the step c was conducted was recorded as day 0.
   d. A cell morphological change was observed daily under a microscope, and culturing was conducted consecutively for 2 d.
   e. On day 3, a cell morphology was observed under a microscope. If the cell morphology changed significantly and a confluency of cells reached 100%, passage was allowed.
   f. When the passage was conducted, 10 µL of a reprogramming additive III with Catalog No. CA5002002-3 was added to 10 mL of the medium from the UrinEasy urine cell expansion culture kit, and thorough mixing was conducted to prepare a reprogramming medium C. A medium in a cell culture well to be digested was discarded, and washing was conducted once with PBS. 50 µL of trypsin was added to the cell culture well, and digestion was conducted in a 37°C incubator for 3 min. 100 µL of the reprogramming medium C was added to terminate the digestion, and a bottom surface of the cell culture well was rinsed. A resulting cell suspension was collected in a centrifuge tube with the reprogramming medium C, and pipetted up and down for thorough mixing. Suspended cells were inoculated evenly into a culture plate coated with Matrigel at a passaging ratio of 1:10.
   g. On day 4, a medium change was conducted with the medium from the UrinEasy urine cell expansion culture kit.
   h. A cell status was monitored daily. If small cluster colonies were observed under a microscope, a medium change was conducted daily using a Reproeasy human cell reprogramming basal medium with Catalog No. CA5003050-1.
   i. If a plurality of clones each composed of 10 or more cells were observed, an original medium was replaced by a PGM1 human PSC medium with Catalog No. CA1007500.
   j. Cells were observed under a microscope. When the plurality of clones grew to fill a field of view under a 10X objective lens, the plurality of clones were cut using a 1 mL syringe needle, and separated from a bottom surface of a culture well using a 200 µL sterile pipette tip. Then, the plurality of clones were transferred to a Matrigel-coated 24-well plate with the PGM1 human PSC medium, and cultured in a 37°C incubator with a CO₂ volume concentration of 5% and a humidity of 90% or more.
   k. Observation was conducted daily, and a medium change was conducted with the PGM1 human PSC medium.
2) The expansion culture of the hiPSC line:
   a. When it was observed under a microscope that cells grew to a confluency of about 80% and were in a prominent status, or when clones were unevenly distributed and individual clones were relatively large, passage was allowed.
   b. A medium in a well was discarded, and washing was conducted once with PBS. A DissoEasy human PSC digestion solution with Catalog No. CA1023100 was added, and digestion was conducted at room temperature for 5 min. The DissoEasy human PSC digestion solution in the well was removed, and the PGM1 human PSC medium was added. A bottom surface of the well was gently rinsed in a fan-shaped pattern to make cells detach, and a resulting cell suspension was collected in a centrifuge tube. The cell suspension was inoculated into a Matrigel-coated culture plate at a volume ratio of 1:10, and cultured in a 37°C incubator with a CO₂ volume concentration of 5% and a humidity of 90% or more. Subsequently, observation was conducted daily, and a medium change was conducted daily with the PGM1 human PSC medium.

(3) Differentiation of the sebaceous gland progenitor cell
a. A basal differentiation medium for hiPSC obtained in the step (2) to differentiate into a sebaceous gland progenitor cell was prepared. An original medium for the hiPSC was replaced with the basal differentiation medium. The basal differentiation medium included the following components:

| | |
|---|---|
| DMEM : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| RA | 1 µM. |

b. A medium change was conducted daily with the basal differentiation medium prepared in the step (a), and culturing was conducted consecutively for 3 d in a 37°C incubator with a CO₂ volume concentration of 5%.
c. After the 3 d culturing, the basal differentiation medium was replaced with a sebaceous gland progenitor cell differentiation activation medium. The sebaceous gland progenitor cell differentiation activation medium was produced by adding an epidermal growth factor at 20 ng/mL and a bone morphogenetic protein 4 at 1 ng/mL to the basal differentiation medium in the step (a).
d. A medium change was conducted with the sebaceous gland progenitor cell differentiation activation medium every other day, and culturing was conducted consecutively for 8 d in a 37°C incubator with a CO₂ volume concentration of 5%.
e. A sebaceous gland progenitor cell differentiation medium was prepared. The sebaceous gland progenitor cell differentiation medium included the following components:
EpiLife^{™} medium : Dermacult keratinocyte expansion medium volume ratio: 1:1

| | |
|---|---|
| epidermal growth factor | 20 ng/mL and |
| bone morphogenetic protein 4 | 1 ng/mL. |

f. The sebaceous gland progenitor cell differentiation activation medium was replaced with the sebaceous gland progenitor cell differentiation medium, and culturing was conducted consecutively for 10 d to 11 d in a 37°C incubator with a CO₂ volume concentration of 5%. Cells were digested with TrypLE in a CO₂ incubator at 37°C for 15 min. Centrifugation was conducted at 200 × g for 3 min, and a resulting supernatant was discarded. Resulting cells were resuspended in the sebaceous gland progenitor cell differentiation medium supplemented with a dihydrochloride ATP-competitive ROCK inhibitor at 10 µM.
g. The cells obtained in the step (f) were inoculated into a culture plate coated with fibronectin at 25 µg/mL.
h. Culturing was conducted consecutively for 13 d in a 37°C incubator with a CO₂ volume concentration of 5%, and an original medium was replaced with a fresh sebaceous gland progenitor cell differentiation medium.
i. A medium change was conducted daily, and culturing was conducted consecutively for 9 d in a 37°C incubator with a CO₂ volume concentration of 5% to produce the sebaceous gland progenitor cell.

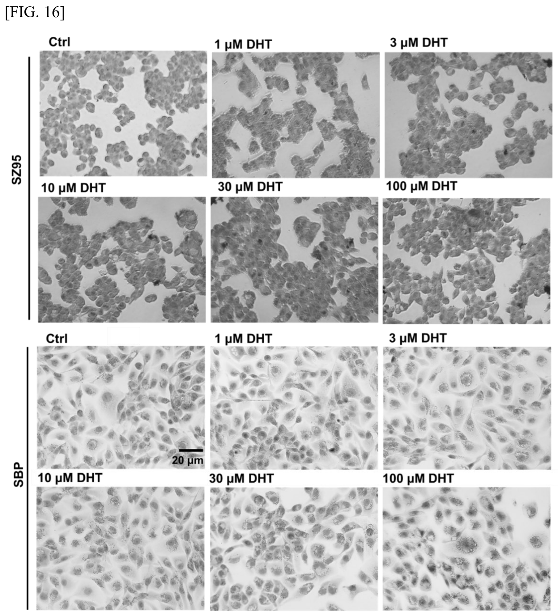

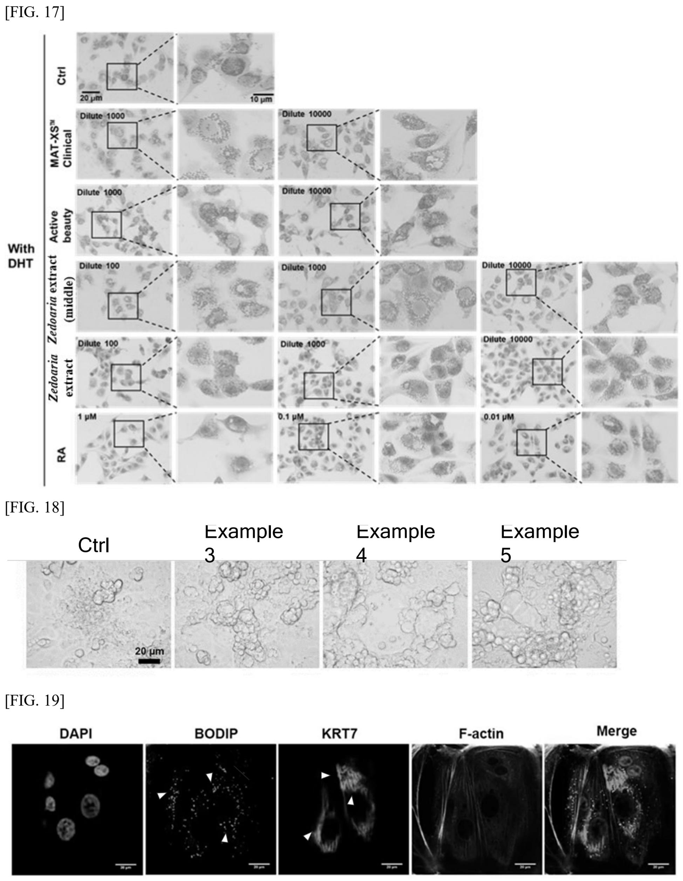

The sebaceous gland progenitor cell obtained in this example was used to evaluate and screen oil-regulating drugs or skincare products:
1. Liquid droplet changes in the sebaceous gland progenitor cell (SBP) and an immortalized human sebaceous gland cell (SZ95) before and after the intervention of DHT at different concentrations
   **a.** Cells were inoculated into a 96-well plate. The intervention was conducted for 24 h with DHT at different concentrations (1 µM, 3 µM, 10 µM, 30 µM, and 100 µM). Then, a culture supernatant was removed. Cells were washed twice with PBS and fixed with 4% paraformaldehyde for 20 min. The fixation solution was removed, and washing was conducted twice with distilled water. A 60% isopropanol solution was added to immerse cells for 15 min, and then discarded. Washing was conducted twice with distilled water. A prepared oil red O staining solution was added, and staining was conducted for 20 min. The oil red O staining solution was removed, and washing was conducted with water until no staining solution remained. Nuclei were counterstained with a hematoxylin staining solution for 2 min. The hematoxylin staining solution was discarded, and washing was conducted with tap water until no staining solution remained. Distilled water was added to cover cells. The cells were observed and photographed under a microscope.
   **b.** As shown in FIG. 16, compared with the immortalized human sebaceous gland cell, the sebaceous gland progenitor cell obtained in this example exhibited a pronounced response to DHT intervention. After the DHT intervention, the liquid droplet volume and number significantly increased. Therefore, the sebaceous gland progenitor cell obtained in this example could be used for subsequent drug or skincare product screening.
2. Liquid droplet changes in DHT-stimulated sebaceous gland progenitor cells (SBP) after the intervention with different drugs and skincare products
   **a.** Cells were inoculated into a 96-well plate. The intervention was conducted for 24 h with DHT at 100 µM. The intervention was further conducted for 24 h with various drugs at different concentrations. A culture supernatant was removed. Cells were washed twice with PBS and fixed with 4% paraformaldehyde for 20 min. The fixation solution was removed, and washing was conducted twice with distilled water. A 60% isopropanol solution was added to immerse cells for 15 min, and then discarded. Washing was conducted twice with distilled water. A prepared oil red O staining solution was added, and staining was conducted for 20 min. The oil red O staining solution was removed, and washing was conducted with water until no staining solution remained. Nuclei were counterstained with a hematoxylin staining solution for 2 min. The hematoxylin staining solution was discarded, and washing was conducted with water until no staining solution remained. Distilled water was added to cover cells. The cells were observed and photographed under a microscope.
   **b.** As shown in FIG. 17, after the sebaceous gland progenitor cell obtained in this example was stimulated with DHT, the intervention of RA, a *Zedoaria* extract, and MAT-XS^{™}-Clinical at different concentrations led to the decline in both the volume and number of liquid droplets generated by the sebaceous gland progenitor cell (SBP) in a concentration-dependent manner. It can be known that the method of the present disclosure can be utilized for screening and evaluating oil-regulating drugs or skincare products.

Example 3: The present disclosure provides an immortalized human sebaceous gland progenitor cell deposited at the China Center for Type Culture Collection (CCTCC) under an accession number of CCTCC NO: C2023286. A preparation method of the immortalized human sebaceous gland progenitor cell includes the following steps:
(1) Isolation and expansion culture of a renal epithelial cell:
1) The isolation of the renal epithelial cell:
   a. Urine was collected from an acne patient in a 50 mL centrifuge tube and centrifuged at 400 × g and room temperature for 10 min. A resulting supernatant was discarded with 1 mL of the supernatant left.
   b. A resulting pellet was resuspended with 10 mL of a washing solution from a UrinEasy urine cell isolation and culture kit, transferred to another centrifuge tube, and centrifuged at 200 × g and room temperature for 10 min. A resulting supernatant was discarded. A resulting cell pellet was resuspended with a medium from the UrinEasy urine cell isolation and culture kit, and inoculated into a culture plate coated with Matrigel in advance.
   c. Culturing was conducted for 24 h, and subsequently, whether there was contamination was observed. If there was no contamination, 800 µL of the medium from the UrinEasy urine cell isolation and culture kit was added, and culturing was further conducted for 3 d.
   d. On day 4, monitoring was conducted for contamination and adherent cells. If there was no contamination, 2/3 of a medium in each well was discarded, and 800 µL of the medium from the UrinEasy urine cell isolation and culture kit was further added. Subsequently, monitoring was conducted daily for contamination and adherent cells, and a partial-medium or complete-medium change was conducted with the medium every other day.
2) The expansion culture of the renal epithelial cell:
   a. After the isolation in the step 1), when a confluency of the renal epithelial cell reached 80%, passage and expansion were allowed.
   b. A medium in each well was discarded, and washing was conducted once with PBS. A trypsin digestion solution at a mass concentration of 0.25% was added, and digestion was conducted in a 37°C incubator for 3 min. An expansion culture basal medium from a UrinEasy urine cell expansion culture kit was added in a volume twice a volume of the trypsin digestion solution to terminate the digestion, and a bottom surface of each culture well was gently rinsed to make cells detach.
   c. A resulting cell suspension was collected in a 15 mL centrifuge tube and centrifuged at 1,200 rpm and room temperature for 3 min. A resulting supernatant was discarded. A resulting cell pellet was resuspended with the expansion culture basal medium from the UrinEasy urine cell expansion culture kit, and gently pipetted up and down to produce a single-cell suspension. The single-cell suspension was inoculated evenly into a culture plate coated with Matrigel.

(2) Establishment and expansion culture of a hiPSC line:
1) The establishment of the hiPSC line:
   a. When the renal epithelial cell derived from the urine in the step (1) was passaged, the renal epithelial cell was inoculated at cell densities of 5,000/well, 10,000/well, and 15,000/well into a 96-well plate coated with Matrigel, with three replicate wells for each density. The day on which the step a was conducted was recorded as day -1.
   b. The next day, a cell status was observed under a microscope, and a well was selected from each density for cell counting. A culture well with a cell count of 10,000 to 20,000 was selected for subsequent experiments.
   c. 10 µL of a reprogramming additive I with Catalog No. CA5002002-1 and 10 mL of a medium from the UrinEasy urine cell expansion culture kit were mixed thoroughly to prepare a reprogramming medium A. 100 µL of the reprogramming medium A and 10 µL of a reprogramming additive II with Catalog No. CA5002002-2 were mixed thoroughly to prepare a reprogramming medium B. A medium in the selected culture well was discarded, and the reprogramming medium B was added. The day on which the step c was conducted was recorded as day 0.
   d. A cell morphological change was observed daily under a microscope, and culturing was conducted consecutively for 2 d.
   e. On day 3, a cell morphology was observed under a microscope. If the cell morphology changed significantly and a confluency of cells reached 100%, passage was allowed.
   f. When the passage was conducted, 10 µL of a reprogramming additive III with Catalog No. CA5002002-3 was added to 10 mL of the medium from the UrinEasy urine cell expansion culture kit, and thorough mixing was conducted to prepare a reprogramming medium C. A medium in a cell culture well to be digested was discarded, and washing was conducted once with PBS. 50 µL of trypsin was added to the cell culture well, and digestion was conducted in a 37°C incubator for 3 min. 100 µL of the reprogramming medium C was added to terminate the digestion, and a bottom surface of the cell culture well was rinsed. A resulting cell suspension was collected in a centrifuge tube with the reprogramming medium C, and pipetted up and down for thorough mixing. Suspended cells were inoculated evenly into a culture plate coated with Matrigel at a passaging ratio of 1:7.
   g. On day 4, a medium change was conducted with the medium from the UrinEasy urine cell expansion culture kit.
   h. A cell status was monitored daily. If small cluster colonies were observed under a microscope, a medium change was conducted daily using a Reproeasy human cell reprogramming basal medium with Catalog No. CA5003050-1.
   i. If a plurality of clones each composed of 10 or more cells were observed, an original medium was replaced by a PGM1 human PSC medium with Catalog No. CA1007500.
   j. Cells were observed under a microscope. When the plurality of clones grew to fill a field of view under a 10X objective lens, the plurality of clones were cut using a 1 mL syringe needle, and separated from a bottom surface of a culture well using a 200 µL sterile pipette tip. Then, the plurality of clones were transferred to a Matrigel-coated 24-well plate with the PGM1 human PSC medium, and cultured in a 37°C incubator with a CO₂ volume concentration of 5% and a humidity of 90% or more.
   k. Observation was conducted daily, and a medium change was conducted with the PGM1 human PSC medium.
2) The expansion culture of the hiPSC line:
   a. When it was observed under a microscope that cells grew to a confluency of about 80% and were in a prominent status, or when clones were unevenly distributed and individual clones were relatively large, passage was allowed.
   b. A medium in a well was discarded, and washing was conducted once with PBS. A DissoEasy human PSC digestion solution with Catalog No. CA1023100 was added, and digestion was conducted at room temperature for 4 min. The DissoEasy human PSC digestion solution in the well was removed, and the PGM1 human PSC medium was added. A bottom surface of the well was gently rinsed in a fan-shaped pattern to make cells detach, and a resulting cell suspension was collected in a centrifuge tube. The cell suspension was inoculated into a Matrigel-coated culture plate at a volume ratio of 1:6, and cultured in a 37°C incubator with a CO₂ volume concentration of 5% and a humidity of 90% or more. Subsequently, observation was conducted daily, and a medium change was conducted daily with the PGM1 human PSC medium.

(3) Differentiation of the sebaceous gland progenitor cell
a. A basal differentiation medium for hiPSC obtained in the step (2) to differentiate into a sebaceous gland progenitor cell was prepared. An original medium for the hiPSC was replaced with the basal differentiation medium. The basal differentiation medium included the following components:

| | |
|---|---|
| DMEM : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| RA | 1 µM. |

b. A medium change was conducted daily with the basal differentiation medium prepared in the step (a), and culturing was conducted consecutively for 3 d in a 37°C incubator with a CO₂ volume concentration of 5%.
c. After the 3 d culturing, the basal differentiation medium was replaced with a sebaceous gland progenitor cell differentiation activation medium. The sebaceous gland progenitor cell differentiation activation medium was produced by adding an epidermal growth factor at 20 ng/mL and a bone morphogenetic protein 4 at 1 ng/mL to the basal differentiation medium in the step (a).
d. A medium change was conducted with the sebaceous gland progenitor cell differentiation activation medium every other day, and culturing was conducted consecutively for 7 d in a 37°C incubator with a CO₂ volume concentration of 5%.
e. A sebaceous gland progenitor cell differentiation medium was prepared. The sebaceous gland progenitor cell differentiation medium included the following components:

| | |
|---|---|
| EpiLife^{™} medium : Dermacult keratinocyte expansion medium | volume ratio: 1:1 |
| epidermal growth factor | 20 ng/mL and |
| bone morphogenetic protein 4 | 1 ng/mL. |

f. The sebaceous gland progenitor cell differentiation activation medium was replaced with the sebaceous gland progenitor cell differentiation medium, and culturing was conducted consecutively for 10 d in a 37°C incubator with a CO₂ volume concentration of 5%. Cells were digested with TrypLE in a CO₂ incubator at 37°C for 15 min. Centrifugation was conducted at 200 × g for 3 min, and a resulting supernatant was discarded. Resulting cells were resuspended in the sebaceous gland progenitor cell differentiation medium supplemented with a dihydrochloride ATP-competitive ROCK inhibitor at 10 µM.
g. The cells obtained in the step (f) were inoculated into a culture plate coated with fibronectin at 25 µg/mL.
h. Culturing was conducted consecutively until day 12 in a 37°C incubator with a CO₂ volume concentration of 5%, and an original medium was replaced with a fresh sebaceous gland progenitor cell differentiation medium.
i. A medium change was conducted daily, and culturing was conducted consecutively for 7 d in a 37°C incubator with a CO₂ volume concentration of 5% to produce the sebaceous gland progenitor cell.

The sebaceous gland progenitor cell was cultured to differentiate into a mature sebaceous gland cell and the mature sebaceous gland cell was then used to screen and evaluate oil-regulating drugs or skincare products. A method for inducing the sebaceous gland progenitor cell to differentiate into the mature sebaceous gland cell was as follows:
**a.** A basal differentiation medium for the mature sebaceous gland cell was prepared. The basal differentiation medium included the following components:

| | |
|---|---|
| DMEM : Ham's F12 | volume ratio: 3:1 |
| fetal bovine serum | mass concentration: 2.5% |
| insulin | 10 ng/mL |
| epidermal growth factor | 3 ng/mL |
| hydrocortisone | 45.2 ng/mL |
| isoprenaline | 1 µM and |
| adenine | 24 µg/mL. |

**b.** The sebaceous gland progenitor cell was subjected to digestion for 15 min with TrypLE in a 37°C incubator with a CO₂ volume concentration of 5%. Centrifugation was conducted at 250 × g for 3 min, and a resulting supernatant was discarded. A dihydrochloride ATP-competitive ROCK inhibitor was added at 10 µM to the basal differentiation medium for the mature sebaceous gland cell, and a resulting medium was used to resuspend a resulting cell pellet. Cells were inoculated into a culture plate with a cell density of 2.5 × 10⁴ cells/cm², and cultured overnight in a 37°C incubator with a CO₂ volume concentration of 5%.
**c.** The next day, a medium change was conducted with the basal differentiation medium for the mature sebaceous gland cell, and a TGF-β/Smad inhibitor was added at 10 µM.
**d.** A medium change was conducted daily, and culturing was conducted consecutively for 3 d in a 37°C incubator with a CO₂ volume concentration of 5%.
**e.** 3 d later, the basal differentiation medium for the mature sebaceous gland cell was replaced with a final differentiation medium for the mature sebaceous gland cell, and culturing was conducted in a 37°C incubator with a CO₂ volume concentration of 5%.
**f.** A medium change was conducted daily, and culturing was conducted consecutively for 3 d in a 37°C incubator with a CO₂ volume concentration of 5% to produce the mature sebaceous gland cell. The mature sebaceous gland cell could be utilized for the screening and evaluation of oil-regulating drugs or skincare products.

The final differentiation medium for the mature sebaceous gland cell in the step (e) was prepared by adding a TGF-β/Smad inhibitor at 10 µM to the basal differentiation medium for the mature sebaceous gland cell.

Example 4: The present disclosure provides an immortalized human sebaceous gland progenitor cell deposited at the China Center for Type Culture Collection (CCTCC) under an accession number of CCTCC NO: C2023286. A preparation method of the immortalized human sebaceous gland progenitor cell includes the following steps:
(1) Isolation and expansion culture of a renal epithelial cell:
1) The isolation of the renal epithelial cell:
   a. Urine was collected from an acne patient in a 50 mL centrifuge tube and centrifuged at 400 × g and room temperature for 10 min. A resulting supernatant was discarded with 2 mL of the supernatant left.
   b. A resulting pellet was resuspended with 10 mL of a washing solution from a UrinEasy urine cell isolation and culture kit, transferred to another centrifuge tube, and centrifuged at 200 × g and room temperature for 10 min. A resulting supernatant was discarded. A resulting cell pellet was resuspended with a medium from the UrinEasy urine cell isolation and culture kit, and inoculated into a culture plate coated with Matrigel in advance.
   c. Culturing was conducted for 24 h, and subsequently, whether there was contamination was observed. If there was no contamination, 800 µL of the medium from the UrinEasy urine cell isolation and culture kit was added, and culturing was further conducted for 3 d.
   d. On day 4, monitoring was conducted for contamination and adherent cells. If there was no contamination, 2/3 of a medium in each well was discarded, and 800 µL of the medium from the UrinEasy urine cell isolation and culture kit was further added. Subsequently, monitoring was conducted daily for contamination and adherent cells, and a partial-medium or complete-medium change was conducted with the medium every other day.
2) The expansion culture of the renal epithelial cell:
   a. After the isolation in the step 1), when a confluency of the renal epithelial cell reached 90%, passage and expansion were allowed.
   b. A medium in each well was discarded, and washing was conducted once with PBS. A trypsin digestion solution at a mass concentration of 0.25% was added, and digestion was conducted in a 37°C incubator for 3 min. An expansion culture basal medium from a UrinEasy urine cell expansion culture kit was added in a volume twice a volume of the trypsin digestion solution to terminate the digestion, and a bottom surface of each culture well was gently rinsed to make cells detach.
   c. A resulting cell suspension was collected in a 15 mL centrifuge tube and centrifuged at 1,200 rpm and room temperature for 3 min. A resulting supernatant was discarded. A resulting cell pellet was resuspended with the expansion culture basal medium from the UrinEasy urine cell expansion culture kit, and gently pipetted up and down to produce a single-cell suspension. The single-cell suspension was inoculated evenly into a culture plate coated with Matrigel.

(2) Establishment and expansion culture of a hiPSC line:
1) The establishment of the hiPSC line:
   a. When the renal epithelial cell derived from the urine in the step (1) was passaged, the renal epithelial cell was inoculated at cell densities of 5,000/well, 10,000/well, and 15,000/well into a 96-well plate coated with Matrigel, with three replicate wells for each density. The day on which the step a was conducted was recorded as day -1.
   b. b. The next day, a cell status was observed under a microscope, and a well was selected from each density for cell counting. A culture well with a cell count of 10,000 to 20,000 was selected for subsequent experiments.
   c. 10 µL of a reprogramming additive I with Catalog No. CA5002002-1 and 10 mL of a medium from the UrinEasy urine cell expansion culture kit were mixed thoroughly to prepare a reprogramming medium A. 100 µL of the reprogramming medium A and 10 µL of a reprogramming additive II with Catalog No. CA5002002-2 were mixed thoroughly to prepare a reprogramming medium B. A medium in the selected culture well was discarded, and the reprogramming medium B was added. The day on which the step c was conducted was recorded as day 0.
   d. A cell morphological change was observed daily under a microscope, and culturing was conducted consecutively for 2 d.
   e. On day 3, a cell morphology was observed under a microscope. If the cell morphology changed significantly and a confluency of cells reached 100%, passage was allowed.
   f. When the passage was conducted, 10 µL of a reprogramming additive III with Catalog No. CA5002002-3 was added to 10 mL of the medium from the UrinEasy urine cell expansion culture kit, and thorough mixing was conducted to prepare a reprogramming medium C. A medium in a cell culture well to be digested was discarded, and washing was conducted once with PBS. 50 µL of trypsin was added to the cell culture well, and digestion was conducted in a 37°C incubator for 3 min. 100 µL of the reprogramming medium C was added to terminate the digestion, and a bottom surface of the cell culture well was rinsed. A resulting cell suspension was collected in a centrifuge tube with the reprogramming medium C, and pipetted up and down for thorough mixing. Suspended cells were inoculated evenly into a culture plate coated with Matrigel at a passaging ratio of 1:8.
   g. On day 4, a medium change was conducted with the medium from the UrinEasy urine cell expansion culture kit.
   h. A cell status was monitored daily. If small cluster colonies were observed under a microscope, a medium change was conducted daily using a Reproeasy human cell reprogramming basal medium with Catalog No. CA5003050-1.
   i. If a plurality of clones each composed of 10 or more cells were observed, an original medium was replaced by a PSCeasy human PSC recovery medium.
   j. Cells were observed under a microscope. When the plurality of clones grew to fill a field of view under a 10X objective lens, the plurality of clones were cut using a 1 mL syringe needle, and separated from a bottom surface of a culture well using a 200 µL sterile pipette tip. Then, the plurality of clones were transferred to a Matrigel-coated 24-well plate with the PGM1 human PSC medium, and cultured in a 37°C incubator with a CO₂ volume concentration of 5% and a humidity of 90% or more.
   k. Observation was conducted daily, and a medium change was conducted with the PGM1 human PSC medium.
2) The expansion culture of the hiPSC line:
   a. When it was observed under a microscope that cells grew to a confluency of about 80% and were in a prominent status, or when clones were unevenly distributed and individual clones were relatively large, passage was allowed.
   b. A medium in a well was discarded, and washing was conducted once with PBS. A DissoEasy human PSC digestion solution with Catalog No. CA1023100 was added, and digestion was conducted at room temperature for 5 min. The DissoEasy human PSC digestion solution in the well was removed, and the PGM1 human PSC medium was added. A bottom surface of the well was gently rinsed in a fan-shaped pattern to make cells detach, and a resulting cell suspension was collected in a centrifuge tube. The cell suspension was inoculated into a Matrigel-coated culture plate at a volume ratio of 1:8, and cultured in a 37°C incubator with a CO₂ volume concentration of 5% and a humidity of 90% or more. Subsequently, observation was conducted daily, and a medium change was conducted daily with the PGM1 human PSC medium.

(3) Differentiation of the sebaceous gland progenitor cell
a. A basal differentiation medium for hiPSC obtained in the step (2) to differentiate into a sebaceous gland progenitor cell was prepared. An original medium for the hiPSC was replaced with the basal differentiation medium. The basal differentiation medium included the following components:

| | |
|---|---|
| DMEM : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| RA | 1 µM. |

b. A medium change was conducted daily with the basal differentiation medium prepared in the step (a), and culturing was conducted consecutively for 3 d in a 37°C incubator with a CO₂ volume concentration of 5%.
c. After the 3 d culturing, the basal differentiation medium was replaced with a sebaceous gland progenitor cell differentiation activation medium. The sebaceous gland progenitor cell differentiation activation medium was produced by adding an epidermal growth factor at 20 ng/mL and a bone morphogenetic protein 4 at 1 ng/mL to the basal differentiation medium in the step (a).
d. A medium change was conducted with the sebaceous gland progenitor cell differentiation activation medium every other day, and culturing was conducted consecutively for 8 d in a 37°C incubator with a CO₂ volume concentration of 5%.
e. A sebaceous gland progenitor cell differentiation medium was prepared. The sebaceous gland progenitor cell differentiation medium included the following components:

| | |
|---|---|
| EpiLife^{™} medium : Dermacult keratinocyte expansion medium | volume ratio: 1:1 |
| epidermal growth factor | 20 ng/mL and |
| bone morphogenetic protein 4 | 1 ng/mL. |

f. The sebaceous gland progenitor cell differentiation activation medium was replaced with the sebaceous gland progenitor cell differentiation medium, and culturing was conducted consecutively for 11 d in a 37°C incubator with a CO₂ volume concentration of 5%. Cells were digested with TrypLE in a CO₂ incubator at 37°C for 15 min. Centrifugation was conducted at 200 × g for 3 min, and a resulting supernatant was discarded. Resulting cells were resuspended in the sebaceous gland progenitor cell differentiation medium supplemented with a dihydrochloride ATP-competitive ROCK inhibitor at 10 µM.
g. The cells obtained in the step (f) were inoculated into a culture plate coated with fibronectin at 25 µg/mL.
h. Culturing was conducted consecutively for 13 d in a 37°C incubator with a CO₂ volume concentration of 5%, and an original medium was replaced with a fresh sebaceous gland progenitor cell differentiation medium.
i. A medium change was conducted daily, and culturing was conducted consecutively for 8 d in a 37°C incubator with a CO₂ volume concentration of 5% to produce the sebaceous gland progenitor cell.

The sebaceous gland progenitor cell was cultured to differentiate into a mature sebaceous gland cell and the mature sebaceous gland cell was then used to screen and evaluate oil-regulating drugs or skincare products. A method for inducing the sebaceous gland progenitor cell to differentiate into the mature sebaceous gland cell was as follows:
**a.** A basal differentiation medium for the mature sebaceous gland cell was prepared. The basal differentiation medium included the following components:

| | |
|---|---|
| DMEM : Ham's F12 | volume ratio: 3:1 |
| fetal bovine serum | mass concentration: 2.5% |
| insulin | 10 ng/mL |
| epidermal growth factor | 3 ng/mL |
| hydrocortisone | 45.2 ng/mL |
| isoprenaline | 1 µM and |
| adenine | 24 µg/mL. |

**b.** The sebaceous gland progenitor cell was subjected to digestion for 15 min with TrypLE in a 37°C incubator with a CO₂ volume concentration of 5%. Centrifugation was conducted at 250 × g for 3 min, and a resulting supernatant was discarded. A dihydrochloride ATP-competitive ROCK inhibitor was added at 10 µM to the basal differentiation medium for the mature sebaceous gland cell, and a resulting medium was used to resuspend a resulting cell pellet. Cells were inoculated into a culture plate with a cell density of 2.5 × 10⁴ cells/cm², and cultured overnight in a 37°C incubator with a CO₂ volume concentration of 5%.
**c.** The next day, a medium change was conducted with the basal differentiation medium for the mature sebaceous gland cell, and a TGF-β/Smad inhibitor was added at 10 µM.
**d.** A medium change was conducted daily, and culturing was conducted consecutively for 3 d in a 37°C incubator with a CO₂ volume concentration of 5%.
**e.** 3 d later, the basal differentiation medium for the mature sebaceous gland cell was replaced with a final differentiation medium for the mature sebaceous gland cell, and culturing was conducted in a 37°C incubator with a CO₂ volume concentration of 5%.
**f.** A medium change was conducted daily, and culturing was conducted consecutively for 6 d in a 37°C incubator with a CO₂ volume concentration of 5% to produce the mature sebaceous gland cell. The mature sebaceous gland cell could be utilized for the screening and evaluation of oil-regulating drugs or skincare products.

The final differentiation medium for the mature sebaceous gland cell in the step (e) was prepared by adding a TGF-β/Smad inhibitor at 10 µM and a PPAR-β/δ agonist at 1 µM to the basal differentiation medium for the mature sebaceous gland cell.

Example 5: The present disclosure provides an immortalized human sebaceous gland progenitor cell deposited at the China Center for Type Culture Collection (CCTCC) under an accession number of CCTCC NO: C2023286. A preparation method of the immortalized human sebaceous gland progenitor cell includes the following steps:
(1) Isolation and expansion culture of a renal epithelial cell:
1) The isolation of the renal epithelial cell:
   a. Urine was collected from an acne patient in a 50 mL centrifuge tube and centrifuged at 400 × g and room temperature for 10 min. A resulting supernatant was discarded with 1 mL of the supernatant left.
   b. A resulting pellet was resuspended with 10 mL of a washing solution from a UrinEasy urine cell isolation and culture kit, transferred to another centrifuge tube, and centrifuged at 200 × g and room temperature for 10 min. A resulting supernatant was discarded. A resulting cell pellet was resuspended with a medium from the UrinEasy urine cell isolation and culture kit, and inoculated into a culture plate coated with Matrigel in advance.
   c. Culturing was conducted for 24 h, and subsequently, whether there was contamination was observed. If there was no contamination, 800 µL of the medium from the UrinEasy urine cell isolation and culture kit was added, and culturing was further conducted for 3 d.
   d. On day 4, monitoring was conducted for contamination and adherent cells. If there was no contamination, 2/3 of a medium in each well was discarded, and 800 µL of the medium from the UrinEasy urine cell isolation and culture kit was further added. Subsequently, monitoring was conducted daily for contamination and adherent cells, and a partial-medium or complete-medium change was conducted with the medium every other day.
2) The expansion culture of the renal epithelial cell:
   a. After the isolation in the step 1), when a confluency of the renal epithelial cell reached 80%, passage and expansion were allowed.
   b. A medium in each well was discarded, and washing was conducted once with PBS. A trypsin digestion solution at a mass concentration of 0.25% was added, and digestion was conducted in a 37°C incubator for 3 min. An expansion culture basal medium from a UrinEasy urine cell expansion culture kit was added in a volume twice a volume of the trypsin digestion solution to terminate the digestion, and a bottom surface of each culture well was gently rinsed to make cells detach.
   c. A resulting cell suspension was collected in a 15 mL centrifuge tube and centrifuged at 1,200 rpm and room temperature for 3 min. A resulting supernatant was discarded. A resulting cell pellet was resuspended with the expansion culture basal medium from the UrinEasy urine cell expansion culture kit, and gently pipetted up and down to produce a single-cell suspension. The single-cell suspension was inoculated evenly into a culture plate coated with Matrigel.

(2) Establishment and expansion culture of a hiPSC line:
1) The establishment of the hiPSC line:
   a. When the renal epithelial cell derived from the urine in the step (1) was passaged, the renal epithelial cell was inoculated at cell densities of 5,000/well, 10,000/well, and 15,000/well into a 96-well plate coated with Matrigel, with three replicate wells for each density. The day on which the step a was conducted was recorded as day -1.
   b. The next day, a cell status was observed under a microscope, and a well was selected from each density for cell counting. A culture well with a cell count of 10,000 to 20,000 was selected for subsequent experiments.
   c. 10 µL of a reprogramming additive I with Catalog No. CA5002002-1 and 10 mL of a medium from the UrinEasy urine cell expansion culture kit were mixed thoroughly to prepare a reprogramming medium A. 100 µL of the reprogramming medium A and 10 µL of a reprogramming additive II with Catalog No. CA5002002-2 were mixed thoroughly to prepare a reprogramming medium B. A medium in the selected culture well was discarded, and the reprogramming medium B was added. The day on which the step c was conducted was recorded as day 0.
   d. A cell morphological change was observed daily under a microscope, and culturing was conducted consecutively for 2 d.
   e. On day 3, a cell morphology was observed under a microscope. If the cell morphology changed significantly and a confluency of cells reached 100%, passage was allowed.
   f. When the passage was conducted, 10 µL of a reprogramming additive III with Catalog No. CA5002002-3 was added to 10 mL of the medium from the UrinEasy urine cell expansion culture kit, and thorough mixing was conducted to prepare a reprogramming medium C. A medium in a cell culture well to be digested was discarded, and washing was conducted once with PBS. 50 µL of trypsin was added to the cell culture well, and digestion was conducted in a 37°C incubator for 3 min. 100 µL of the reprogramming medium C was added to terminate the digestion, and a bottom surface of the cell culture well was rinsed. A resulting cell suspension was collected in a centrifuge tube with the reprogramming medium C, and pipetted up and down for thorough mixing. Suspended cells were inoculated evenly into a culture plate coated with Matrigel at a passaging ratio of 1:9.
   g. On day 4, a medium change was conducted with the medium from the UrinEasy urine cell expansion culture kit.
   h. A cell status was monitored daily. If small cluster colonies were observed under a microscope, a medium change was conducted daily using a Reproeasy human cell reprogramming basal medium with Catalog No. CA5003050-1.
   i. If a plurality of clones each composed of 10 or more cells were observed, an original medium was replaced by a PGM1 human PSC medium with Catalog No. CA1007500.
   j. Cells were observed under a microscope. When the plurality of clones grew to fill a field of view under a 10X objective lens, the plurality of clones were cut using a 1 mL syringe needle, and separated from a bottom surface of a culture well using a 200 µL sterile pipette tip. Then, the plurality of clones were transferred to a Matrigel-coated 24-well plate with the PGM1 human PSC medium, and cultured in a 37°C incubator with a CO₂ volume concentration of 5% and a humidity of 90% or more.
   k. Observation was conducted daily, and a medium change was conducted with the PGM1 human PSC medium.
2) The expansion culture of the hiPSC line:
   a. When it was observed under a microscope that cells grew to a confluency of about 80% and were in a prominent status, or when clones were unevenly distributed and individual clones were relatively large, passage was allowed.
   b. A medium in a well was discarded, and washing was conducted once with PBS. A DissoEasy human PSC digestion solution with Catalog No. CA1023100 was added, and digestion was conducted at room temperature for 3 min. The DissoEasy human PSC digestion solution in the well was removed, and the PGM1 human PSC medium was added. A bottom surface of the well was gently rinsed in a fan-shaped pattern to make cells detach, and a resulting cell suspension was collected in a centrifuge tube. The cell suspension was inoculated into a Matrigel-coated culture plate at a volume ratio of 1:10, and cultured in a 37°C incubator with a CO₂ volume concentration of 5% and a humidity of 90% or more. Subsequently, observation was conducted daily, and a medium change was conducted daily with the PGM1 human PSC medium.

(3) Differentiation of the sebaceous gland progenitor cell
a. A basal differentiation medium for hiPSC obtained in the step (2) to differentiate into a sebaceous gland progenitor cell was prepared. An original medium for the hiPSC was replaced with the basal differentiation medium. The basal differentiation medium included the following components:

| | |
|---|---|
| DMEM : Ham's F12 | volume ratio: 3:1 |
| fetal bovine serum | mass concentration: 2% |
| insulin | 5 µg/mL |
| hydrocortisone | 0.5 µg/mL |
| isoprenaline | 1 µM |
| triiodothyronine | 1.37 ng/mL |
| adenine | 24 µg/mL |
| ascorbic acid | 300 µM and |
| RA | 1 µM. |

b. A medium change was conducted daily with the basal differentiation medium prepared in the step (a), and culturing was conducted consecutively for 3 d in a 37°C incubator with a CO₂ volume concentration of 5%.
c. After the 3 d culturing, the basal differentiation medium was replaced with a sebaceous gland progenitor cell differentiation activation medium. The sebaceous gland progenitor cell differentiation activation medium was produced by adding an epidermal growth factor at 20 ng/mL and a bone morphogenetic protein 4 at 1 ng/mL to the basal differentiation medium in the step (a).
d. A medium change was conducted with the sebaceous gland progenitor cell differentiation activation medium every other day, and culturing was conducted consecutively for 7 d in a 37°C incubator with a CO₂ volume concentration of 5%.
e. A sebaceous gland progenitor cell differentiation medium was prepared. The sebaceous gland progenitor cell differentiation medium included the following components:

| | |
|---|---|
| EpiLife^{™} medium : Dermacult keratinocyte expansion medium | volume ratio: 1:1 |
| epidermal growth factor | 20 ng/mL and |
| bone morphogenetic protein 4 | 1 ng/mL. |

f. The sebaceous gland progenitor cell differentiation activation medium was replaced with the sebaceous gland progenitor cell differentiation medium, and culturing was conducted consecutively for 10 d in a 37°C incubator with a CO₂ volume concentration of 5%. Cells were digested with TrypLE in a CO₂ incubator at 37°C for 15 min. Centrifugation was conducted at 200 × g for 3 min, and a resulting supernatant was discarded. Resulting cells were resuspended in the sebaceous gland progenitor cell differentiation medium supplemented with a dihydrochloride ATP-competitive ROCK inhibitor at 10 µM.
g. The cells obtained in the step (f) were inoculated into a culture plate coated with fibronectin at 25 µg/mL.
h. Culturing was conducted consecutively until day 12 in a 37°C incubator with a CO₂ volume concentration of 5%, and an original medium was replaced with a fresh sebaceous gland progenitor cell differentiation medium.
i. A medium change was conducted daily, and culturing was conducted consecutively for 7 d in a 37°C incubator with a CO₂ volume concentration of 5% to produce the sebaceous gland progenitor cell.

The sebaceous gland progenitor cell was cultured to differentiate into a mature sebaceous gland cell and the mature sebaceous gland cell was then used to screen and evaluate oil-regulating drugs or skincare products. A method for inducing the sebaceous gland progenitor cell to differentiate into the mature sebaceous gland cell was as follows:
a. A basal differentiation medium for the mature sebaceous gland cell was prepared. The basal differentiation medium included the following components:

| | |
|---|---|
| DMEM : Ham's F12 | volume ratio: 3:1 |
| fetal bovine serum | mass concentration: 2.5% |
| insulin | 10 ng/mL |
| epidermal growth factor | 3 ng/mL |
| hydrocortisone | 45.2 ng/mL |
| isoprenaline | 1 µM and |
| adenine | 24 µg/mL. |

**b.** The sebaceous gland progenitor cell was subjected to digestion for 15 min with TrypLE in a 37°C incubator with a CO₂ volume concentration of 5%. Centrifugation was conducted at 250 × g for 3 min, and a resulting supernatant was discarded. A dihydrochloride ATP-competitive ROCK inhibitor was added at 10 µM to the basal differentiation medium for the mature sebaceous gland cell, and a resulting medium was used to resuspend a resulting cell pellet. Cells were inoculated into a culture plate with a cell density of 2.5 × 10⁴ cells/cm², and cultured overnight in a 37°C incubator with a CO₂ volume concentration of 5%.
**c.** The next day, a medium change was conducted with the basal differentiation medium for the mature sebaceous gland cell, and a TGF-β/Smad inhibitor was added at 10 µM.
**d.** A medium change was conducted daily, and culturing was conducted consecutively for 3 d in a 37°C incubator with a CO₂ volume concentration of 5%.
**e.** 3 d later, the basal differentiation medium for the mature sebaceous gland cell was replaced with a final differentiation medium for the mature sebaceous gland cell, and culturing was conducted in a 37°C incubator with a CO₂ volume concentration of 5%.
**f.** A medium change was conducted daily, and culturing was conducted consecutively for 5 d in a 37°C incubator with a CO₂ volume concentration of 5% to produce the mature sebaceous gland cell. The mature sebaceous gland cell could be utilized for the screening and evaluation of oil-regulating drugs or skincare products.

The final differentiation medium for the mature sebaceous gland cell in the step (e) was prepared by adding a TGF-β/Smad inhibitor at 10 µM, a GW0742PPAR-β/δ agonist at 1 µM, and cyclopamine at 5 µM to the basal differentiation medium for the mature sebaceous gland cell.

It has been proved through the following experiments that the mature sebaceous gland cells obtained in Examples 3 to 5 of the present disclosure are stable and demonstrate the properties of mature sebaceous gland cells *in vitro.* Thus, the mature sebaceous gland cells can be well used for screening and evaluating oil-regulating drugs or skincare products.

### 1) Morphological observation of the mature sebaceous gland cells obtained in Examples 3 to 5 of the present disclosure

As shown in FIG. 18, morphologies of the mature sebaceous gland cells obtained in Examples 3 to 5 were observed and photographed under an optical microscope. It could be observed that both the number and size of round cells increased (scale = 20 µm).

### 2) BODIP and KRT7 fluorescence staining of the mature sebaceous gland cell obtained in Example 3 of the present disclosure

**a.** Cells were inoculated into an ibidi chambered 8-well slide and cultured. A resulting culture supernatant was discarded. Cells were washed twice with PBS and then fixed with 4% paraformaldehyde for 10 min. The 4% paraformaldehyde was then removed, and washing was conducted twice with PBS. A 1% BSA solution was added, and blocking was conducted at room temperature for 1 h. A primary antibody proportionally diluted (KRT7-mouse; ab216016; diluted at 1:100) was added, and incubation was conducted overnight at 4°C. The next day, the slide was taken out, a liquid in each well was discarded, and washing was conducted three times with PBS. A secondary antibody proportionally diluted (mouse 568; diluted at 1:1,000) was added, and incubation was conducted at room temperature for 1 h. A liquid in each well was discarded, and washing was conducted three times with PBS. Then, BODIP (diluted at 1:1,000) & F-actin (phalloidin 633, diluted at 1:1,000) were added, and incubation was conducted at room temperature for 30 min. A liquid in each well was discarded, and washing was conducted three times with PBS. Then, DAPI (diluted at 1:1,000) was added, and incubation was conducted at room temperature for 15 min. A liquid in each well was discarded, and washing was conducted three times with PBS. Images were captured by a confocal laser scanning microscope. Different fluorescence staining images were merged.
**b.** As shown in FIG. 19, the mature sebaceous gland cell was subjected to fluorescence staining with BODIPY. It was found that the mature sebaceous gland cell obtained in this example exhibited the characteristic of producing or accumulating liquid droplets like sebaceous gland cells in the human. Further, since KRT7 was highly expressed in sebaceous gland cells, the expression of the KRT7 protein in the mature sebaceous gland cell was detected through immunofluorescence staining. It was found that the mature sebaceous gland cell obtained in this example expressed KRT7. Arrows in the figure indicated liquid droplet and KRT7 expression (scale = 20 µm).

### 3) Induced differentiation of mature sebaceous gland cells under varying conditions and responses of the mature sebaceous gland cells to an androgen (DHT):

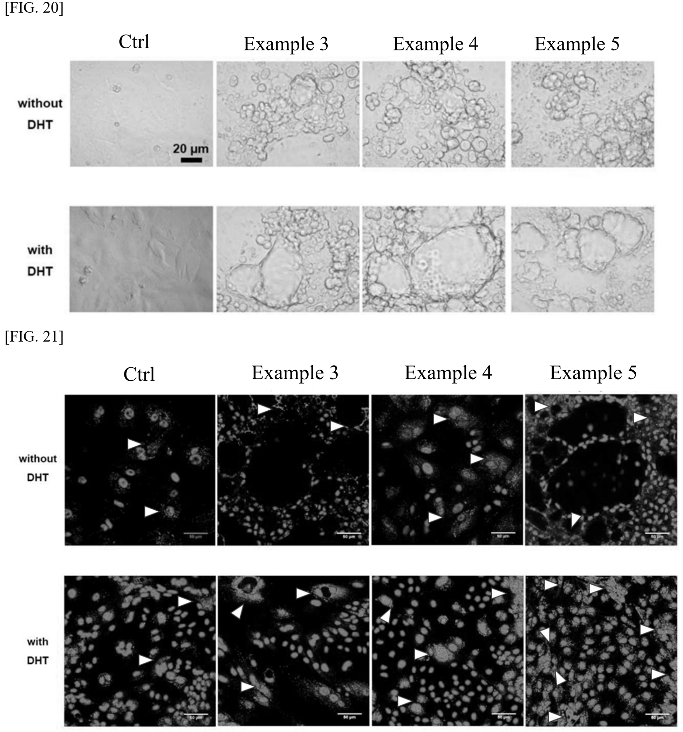

As shown in FIG. 20, when stimulated with DHT, the mature sebaceous gland cells obtained in Examples 3 to 5 underwent accelerated maturation and differentiation and even rupture to release increased liquid droplets/sebum (scale = 20 µm).

### 4) BODIP staining of liquid droplets generated by sebaceous gland cells

**a.** Cells were inoculated into an ibidi chambered 8-well slide. An experimental group was treated with 100 µM DHT (with DHT), and a control group was cultured normally (without DHT) for 24 h. A resulting culture supernatant was discarded. Cells were washed twice with PBS and then fixed with 4% paraformaldehyde for 10 min. The 4% paraformaldehyde was discarded, and washing was conducted twice with PBS. BODIBY (diluted at 1:1,000) was added, and incubation was conducted at room temperature for 30 min. A liquid in each well was discarded, and washing was conducted three times with PBS. DAPI (diluted at 1:1,000) was added, and incubation was conducted at room temperature for 15 min. A liquid in each well was discarded, and washing was conducted three times with PBS. Images were captured by a confocal laser scanning microscope.
**b.** As shown in FIG. 21, responses of the mature sebaceous gland cells obtained in Examples 3 to 5 to DHT were monitored through BODIP staining. When stimulated with DHT, the mature sebaceous gland cells underwent accelerated maturation and differentiation to release increased liquid droplets. In this figure, arrows indicated liquid droplet expression (scale = 20 µm).

### 5) Evaluation of screening effects of mature sebaceous gland cells (SBM) for drugs and skincare products by oil red O staining

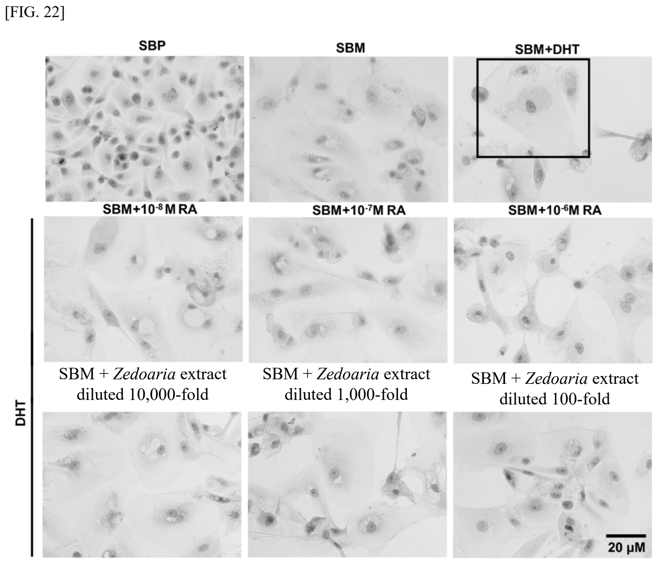

a. The mature sebaceous gland cells were inoculated into a 24-well plate and cultured. When a cell confluency reached 50%, staining was allowed. Cells were washed twice with PBS and fixed with 4% paraformaldehyde for 20 min. The fixation solution was removed, and washing was conducted twice with distilled water. A 60% isopropanol solution was added to immerse cells for 15 min, and then discarded. Washing was conducted twice with distilled water. A prepared oil red O staining solution was added, and staining was conducted for 20 min. The oil red O staining solution was removed, and washing was conducted with water until no staining solution remained. Nuclei were counterstained with a hematoxylin staining solution for 2 min. The hematoxylin staining solution was discarded, and washing was conducted with water until no staining solution remained. Distilled water was added to cover cells. The cells were observed and photographed under a microscope.
b. As shown in FIG. 22, after DHT intervention, the mature sebaceous gland cell (SBM) obtained in this example was irregularly enlarged. After the intervention with RA and a *Zedoaria* extract at different concentrations, the mature sebaceous gland cell (SBM) obtained in this example underwent volume reduction and maturation deceleration according to oil red O staining results, which interfered with the liquid droplet release (scale = 20 µm). Consequently, the mature sebaceous gland cell could be used to evaluate and screen drugs that inhibit sebum secretion or skincare products.

According to the above results, compared to a human keratinocyte, an immortalized human sebaceous gland cell, and hiPSC, the sebaceous gland progenitor cell obtained in the present disclosure includes abundant liquid droplets, can further differentiate into a mature sebaceous gland cell, possesses well-defined genetic information and clinical background, and can simulate behaviors of sebaceous glands in acne patients. Both the sebaceous gland progenitor cell and the mature sebaceous gland cell can be used for evaluation and screening of sebum secretion promotion and inhibition. This method can be used to provide relevant *in vitro* models to enable disease modeling and develop innovative treatments, and can also be used to offer *in vitro* models for the screening of skincare products. It should be noted that, for the sake of brevity, extensive parallel experiments have not been included in this description. In practice, the present disclosure has carried out a plurality of parallel experiments. The mature sebaceous gland cell produced accordingly is also stable and reliable. Moreover, the mature sebaceous gland cells obtained in Examples 3 to 5 of the present disclosure have been individually validated.

The preferred implementations of the present disclosure are described in detail above, but the present disclosure is not limited thereto. Within the scope of the technical concept of the present disclosure, a variety of simple modifications can be made to the technical solutions of the present disclosure, including the combination of specific technical features in any suitable manner. In order to avoid unnecessary repetition, the present disclosure will not describe various possible combinations. However, these simple modifications and combinations should also be regarded as the contents disclosed in the present disclosure, which all fall within the protection scope of the present disclosure.

## Claims

1. An immortalized human sebaceous gland progenitor cell, wherein the immortalized human sebaceous gland progenitor cell is deposited at the China Center for Type Culture Collection (CCTCC) with an accession number of CCTCC NO: C2023286.

2. A method for screening and evaluating oil-regulating drugs or skincare products using the immortalized human sebaceous gland progenitor cell according to claim 1, comprising: directly co-incubating the sebaceous gland progenitor cell with dihydrotestosterone (DHT) at room temperature for 48 h to stimulate the sebaceous gland progenitor cell to generate increased lipids, thereby enabling the screening and evaluating of the oil-regulating drugs or the skincare products; alternatively, culturing the sebaceous gland progenitor cell to differentiate into a mature sebaceous gland cell, and using the mature sebaceous gland cell to screen and evaluate the oil-regulating drugs or the skincare products.

3. The method according to claim 2, wherein when the sebaceous gland progenitor cell is cultured to differentiate into the mature sebaceous gland cell and the mature sebaceous gland cell is then used to screen and evaluate the oil-regulating drugs or the skincare products, a method for inducing the sebaceous gland progenitor cell to differentiate into the mature sebaceous gland cell is as follows:
a. preparing a basal differentiation medium for the mature sebaceous gland cell, wherein the basal differentiation medium comprises the following components:
| | |
|---|---|
| DMEM : Ham's F12 | volume ratio: 3:1 |
| fetal bovine serum | mass concentration: 2.5% |
| insulin | 10 ng/mL |
| epidermal growth factor | 3 ng/mL |
| hydrocortisone | 45.2 ng/mL |
| isoprenaline | 1 µM and |
| adenine | 24 µg/mL; |
b. subjecting the sebaceous gland progenitor cell to digestion for 15 min with TrypLE in a 37°C incubator with a CO₂ volume concentration of 5%; conducting centrifugation at 250 × g for 3 min, and discarding a resulting supernatant; adding a dihydrochloride ATP-competitive ROCK inhibitor at 10 µM to the basal differentiation medium for the mature sebaceous gland cell, and using a resulting medium to resuspend a resulting cell pellet; and inoculating into a culture plate with a cell density of 2.5 × 10⁴ cells/cm², and culturing overnight in a 37°C incubator with a CO₂ volume concentration of 5%;
c. the next day, conducting a medium change with the basal differentiation medium for the mature sebaceous gland cell, and adding a TGF-β/Smad inhibitor at 10 µM;
d. conducting a medium change daily, and culturing consecutively for 3 d in a 37°C incubator with a CO₂ volume concentration of 5%;
e. 3 d later, replacing the basal differentiation medium for the mature sebaceous gland cell with a final differentiation medium for the mature sebaceous gland cell, and culturing in a 37°C incubator with a CO₂ volume concentration of 5%; and
f. conducting a medium change daily, and culturing consecutively for 3 d to 6 d in a 37°C incubator with a CO₂ volume concentration of 5% to produce the mature sebaceous gland cell.

4. The method according to claim 3, wherein the final differentiation medium for the mature sebaceous gland cell in the step (e) is prepared by adding a TGF-β/Smad inhibitor at 10 µM to the basal differentiation medium for the mature sebaceous gland cell; or is prepared by adding the TGF-β/Smad inhibitor at 10 µM and a PPAR-β/δ agonist at 1 µM to the basal differentiation medium for the mature sebaceous gland cell; or is prepared by adding the TGF-β/Smad inhibitor at 10 µM, a GW0742 PPAR-β/δ agonist at 1 µM, and cyclopamine at 5 µM to the basal differentiation medium for the mature sebaceous gland cell.

## Amended claims

### Amended claims under Art. 19.1 PCT

1. A human sebaceous gland progenitor cell, wherein the human sebaceous gland progenitor cell is deposited at China Center for Type Culture Collection (CCTCC) under an accession number of CCTCC NO: C2023286.

2. A method for screening oil-regulating drugs or skincare products using the human sebaceous gland progenitor cell according to claim 1, comprising: directly co-incubating the human sebaceous gland progenitor cell with dihydrotestosterone (DHT) at room temperature for 48 h to stimulate the human sebaceous gland progenitor cell to produce increased lipids, thereby enabling the screening of the oil-regulating drugs or the skincare products.

3. A use of the human sebaceous gland progenitor cell according to claim 1 in screening oil-regulating drugs or skincare products, comprising: culturing the human sebaceous gland progenitor cell to differentiate into a mature sebaceous gland cell, and using the mature sebaceous gland cell to screen the oil-regulating drugs or the skincare products.
